Europäisches Patentamt

European Patent Office

Office européen des brevets

(11) Publication number: **0 230 222**
**A1**

# EUROPEAN PATENT APPLICATION

(21) Application number: 87100064.2

(22) Date of filing: 05.01.87

(51) Int. Cl.⁴: **C12N 15/00** , C07K 13/00 ,
C12N 1/18 , A61K 39/21 ,
C12P 21/02 , G01N 33/569 ,
C07K 15/00

(30) Priority: 06.01.86 US 816645

(43) Date of publication of application:
**29.07.87 Bulletin 87/31**

(84) Designated Contracting States:
**AT BE CH DE ES FR GB IT LI NL SE**

(71) Applicant: **F. HOFFMANN-LA ROCHE & CO.
Aktiengesellschaft**

**CH-4002 Basel(CH)**

(72) Inventor: **Kramer, Richard
9 North Edgewood Avenue
West Orange, N.J. 07052(US)**
Inventor: **Reddy, Premkumar
51 South Mountain Avenue
Montclair, N.J. 07042(US)**
Inventor: **Shaber, Michael
43 Mereline Avenue
West Paterson, N.J. 07424(US)**

(74) Representative: **Lederer, Franz, Dr. et al
Patentanwält Dr. Franz Lederer Lucile
Grahnstrasse 22
D-8000 München 80(DE)**

(54) **Expression of HTLV-III gag-Gene.**

(57) A recombinant gag-protein of the etiologic agent
of acquired immune deficiency syndrome (AIDS) and
the proteolytic proteins produced therefrom as well
as corresponding vectors and transformants express-
ing these proteins are disclosed. In addition, a meth-
od of testing human blood for presence of antibodies
to the AIDS virus using the recombinant gag-protein
or any of its proteolytic proteins or mixtures thereof
is disclosed.

Figure 1-B

BamH I  Aha III          Pst I

Aha III
ACCA ATG TTT AAA
TGGT TAC AAA TTT
Met Phe Lys          *PHO5*

EcoR I          Cla I          Bgl II  Bcl I          EcoR I

36bp  Cla I
ATG ·········· GAT CGA TGG
TAC ·········· CTA GCT ACC
Met ·········· Asp Arg Trp          *gag/pol*

BamHI/Aha III
Isolate 560bp fragment

Cla I
Klenow
EcoR I
Isolate 3900bp fragment

Ligate
BamH I/EcoR I

BamH I          Bgl II  Bcl I          EcoR I

ACCA ATG TTT CGA TGG
Met Phe Arg Trp

## Expression of HTLV-III gag-Gene

### Background of the Invention

The retrovirus HTLV-III and the closely related variants of this virus. LAV and ARV, appear to be the causative agents of the disease Acquired Immunodeficiency Syndrome (AIDS) [see Barré-Sinoussi et al., Science 220, 868-871 (1983); Levy et al., Science 225, 840-842 (1984); Montagnier et al., in Human T-Cell Leukemia/Lymphoma Virus, R. C. Gallo, M. Essex and L. Gross, eds. (Cold Spring Harbor, New York: Cold Spring Harbor Laboratory) pp. 363-370 (1984); Popovic et al., Science 224 . 497-500 (1984): Gallo et al., Science, 224 500-503 (1984); Schüpbach et al., Science 224, 503-505 - (1984)]. There is strong correlation between AIDS and the presence of antibodies to HTLV-III. Furthermore, 85-95% of patients with lymphadenopathy syndrome and a significant proportion of asymptomatic homosexual men in AIDS endemic areas carry circulating antibodies to HTLV-III [see Schüpbach et al., supra]. HTLV-III antibodies have also been widely detected in patients who were exposed to this disease through intravenous drug injections with contaminated needles and in hemophiliacs who received intravenous blood products. Current estimates indicate that approximately one million Americans have been infected with this virus, and approximately 10% of this infected population is expected to acquire this lethal disease.

Molecular cloning and nucleotide sequence analysis of HTLV-III and its variants have demonstrated that this viral genome exhibits many of the structural features of the avian and mammalian retroviruses [see Ratner et al., Nature 313, 277-284 (1985): Sanchez-Pescador et al., Science 227, 484-492 (1985); Wain-Hobson, et al., Cell 40, 9-17 (1985); and Muesing, M. et al., Nature 313, 450-458 (1985)]. Thus, the viral genome contains the three genes - (gag, pol and env) characteristic of all retroviruses. In addition, the HTLV-III genome contains two short open reading frames whose function are unknown.

Effective containment of AIDS depends on development of sensitive and rapid methods to identify individuals exposed to or infected with HTLV-III and therapeutic agents that interfere with viral replication. One of the viral genes, gag, encodes a precursor which is proteolytically processed into core proteins during virion maturation. From DNA sequence data and analysis of isolated viral proteins it follows that the HTLV-III gag precursor comprises about 56 kd and is processed into species of approximately 24, 16, and 14 kd (Ratner et al., supra; Sanchez-Pescador et al., supra; Wain-Hobson et al., supra; and Muesing et al., supra). The protease responsible for this processing is typically encoded by the retroviral genome. It is included in the 3' end of the gag gene in avian retroviruses and in the 5' end of the pol gene in mammalian viruses [for review see Dickson et al., "Protein Biosynthesis and Assembly", in Molecular Biology of Tumor Viruses, R. A. Weiss, N. M. Teich, H. E. Varmus and J. M. Coffin, eds. (Cold Spring Harbor Laboratory: Cold Spring Harbor, NY) pp. 513-648 (1982)]. In at least one mammalian retrovirus, Moloney murine leukemia virus (MuLV), the protease is a gag-pol read-through product. A therapeutic agent that could inhibit this protease might block virus spread. It is, therefore, important to identify the region of the HTLV-III genome that encodes this protease and to develop an in vitro system in which the proteolysis of the gag gene precursor can be studied.

HTLV-III genomes have been molecularly cloned. Shaw et al., Science 226, 1165-1171 (1984). Also the complete nucleotide sequence of the proviral genome of HTLV III has been determined [Ratner et al., supra: and Sanchez-Pescador, et al., supra].

One reason for the difficulty in determining the etiologic agent of AIDS was due to the reactivity of various retroviral antigens with serum samples from AIDS Patients. For example, serum samples from AIDS patients have been shown to react with antigens of HTLV I and HTLV III (HTLV-I: Essex et al., "Antibodies to Cell Membrane Antigens Associated with Human T-Cell Leukemia Virus in Patients with AIDS", Science 220, 859-862 (1983); HTLV-III: Sarngadharan et al., "Antibodies Reactive With Human T-Lymphotropic Retroviruses (HTLV-III) in the Serum of Patients With AIDS", Science 224, 506-508 (1984)). Gene products of HTLV demonstrated antigenicities cross-reactive with antibodies in sera from adult T-cell leukemia patients [Kiyokawa, T. et al., "Envelope proteins of human T-cell leukemia virus: Expression in Escherichia coli and its application to studies of env gene functions", PNAS - (USA) 81, 6202-6206 (1984)]. Adult T-cell leukemias (ATL) differ from acquired immune deficiency syndrome (AIDS) in that HTLV-I causes T-cell malignancies, that is uncontrolled growth of T-cell. In AIDS rather than cell growth there is cell death. In fact this cytopathic characteristic of HTLV III was critical to determining ultimately the specific retroviral origin of the disease. Thus the etiologic agent of AIDS was isolated by use of immortalized human neoplastic T-cell lines (HT) infected with the cytopathic retrovirus characteristic of AIDS, isolated from AIDS afflicted patients. Seroepidemiological assays using this virus showed a complete correlation between AIDS and the presence of antibodies to HTLV III antigens [Sarngadharan et al., supra - (1984); Schüpbach et al., supra]. In addition, nearly 85% of patients with lymphadenopathy syndrome and a significant proportion of asymptomatic homosexual men in AIDS endemic areas were also found to carry circulating antibodies to HTLV III. Taken together, all these data indicate HTLV III to be the etiologic agent for AIDS.

Until the successful culturing of AIDS virus using H-9 cell line the gag AIDS protein of the AIDS virus had not been isolated, characterized or synthesized. This in major part is due to the fact that the virus is cytopathic and thus isolation of the virus was not possible [Popovic, M. et al., supra]. Once the human T-cell line resistant to the cytopathic effects of the virus was discovered, a molecular clone of proviral DNA could be achieved.

The need for a sensitive and rapid method for the diagnosis of AIDS in human blood and its prevention by vaccination is very great. Virtually all the assays/tests presently available are fraught with errors. In fact the Center for Disease Control - (CDC) has indicated that presently available tests be used solely for screening units of blood for antibody to HTLV III. The CDC went further by stating that the presently available ELISA tests can not be used for general screening of high risk populations or as a diagnostic test for AIDS [Federal Register 50(48), 9909, March 12, 1985]. The errors have been traced to the failure to use a specific antigenic protein of the etiologic agent for AIDS. The previously used proteins were derived from a viral lysate. Since the lysate is made from human cells infected with the virus, i.e. the cells used to grow the virus, the lysate will contain human proteins as well as viral proteins. Thus preparation of a pure antigen of viral protein is very difficult. The antigen used produced both false positive and false negative results [Budiansky. S., AIDS Screening, False Test Results Raise Doubts, Nature 312. 583(1984)]. The errors caused by the use of such lysate proteins/peptides can be avoided by using a composition for binding AIDS antibodies which is substantially free of the non-AIDS specific proteins. Compositions that are substantially pure AIDS gag-proteins can be used as antigens.

## Summary of Invention

In accordance with this invention we have produced by recombinant technology the immunologically active portion of the precursor of the HTLV-III gag protein as well as the immunological active portion of the natural proteolytic precusor proteins which result from this gag protein - (hereinafter also referred to as polypeptides immunologically equivalent to the gag-protein products of HTLV-III). In addition, we have produced a recombinant organism capable of expressing these proteins/polypeptides by utilizing various expression vectors capable of expressing all of these proteins. By recombinant technology in accordance with this invention, one produces the precursor gag 56 kd protein with modification resulting from the removal of N-terminal codons at its amino terminus, but having the same immunological activity as the natural precursor gag protein. In view of this modification in the precusor, the 14 kd gag protein which is proteolytically produced therefrom is also modified at its amino terminus from the natural 14 kd gag protein. However, this modified gag protein also has the same immunological activity as its natural form. Also in accordance with this invention, a new p48 protein is produced having the same immunological activity of the natural precursor gag protein. The 16 kd proteolytic protein produced by the process of this invention has a varient in its amino acid structure from the proteolytic 16 kd gag proteins reported in Shaw et al., supra. The 24 kd proteolytic protein is the same as the naturally occuring 24 kd proteolytic protein.

The 56 kd, 24, 16 and 14 kd gag proteins produced by this invention have the same immunological activity as their corresponding natural gag proteins. They have the same epitopes to react with the same antibodies as their corresponding natural proteins.

In accordance with this invention, recombinant DNA techniques are utilized for producing the HTLV-III gag protein having 56 kd as well as the proteolytic proteins produced therefrom, i.e. the proteins having 24,16 and 14 kd, respectively. In the first step of this invention, one isolates from the known genome for the retrovirus HTLV-III a portion which contains the DNA sequence encoding for the gag protein of HTLV-III and this portion is constructed into a gene which contains this DNA sequence encoding for the gag protein of HLTV-III operably linked to a promotor capable of effecting the expression of said DNA sequence and this gene is inserted into an expression vector or plasmid and such vector or plasmid is inserted into a suitable microorganism, preferably a yeast cell, to produce a microorganism capable of expressing the active portion of the gag-protein of HTLV-III. In accordance with this invention, the recombinant organism not only expresses a protein which is immunological equivalent to the natural precursor gag protein, but also expresses the species proteins which are processed by proteolytic enzymes expressed with the precusor protein.

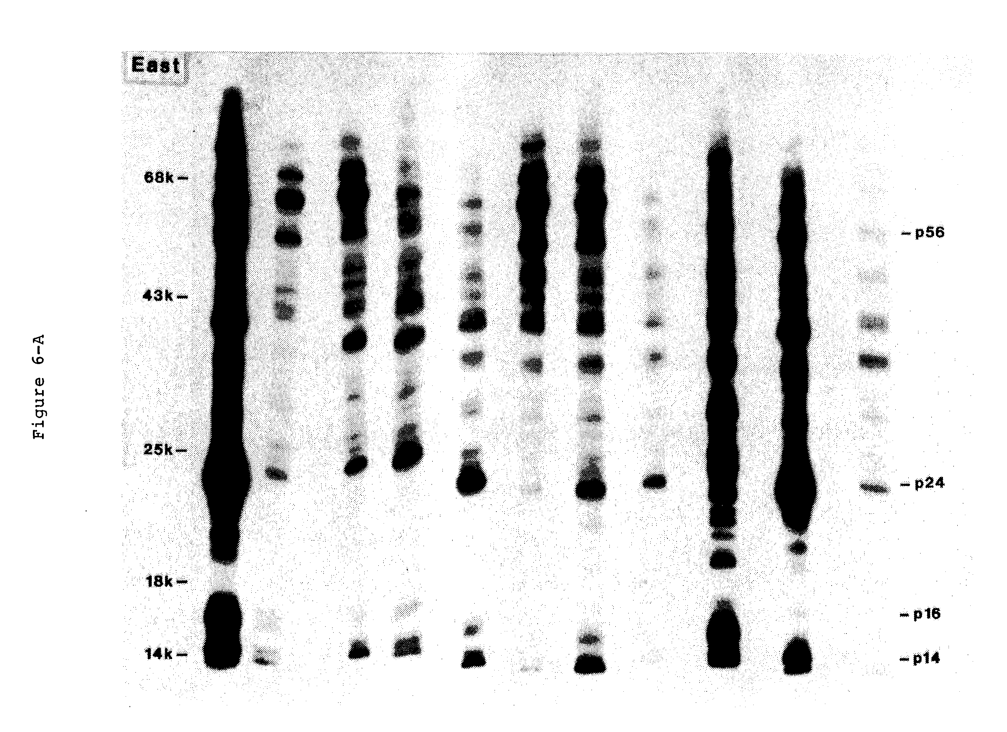

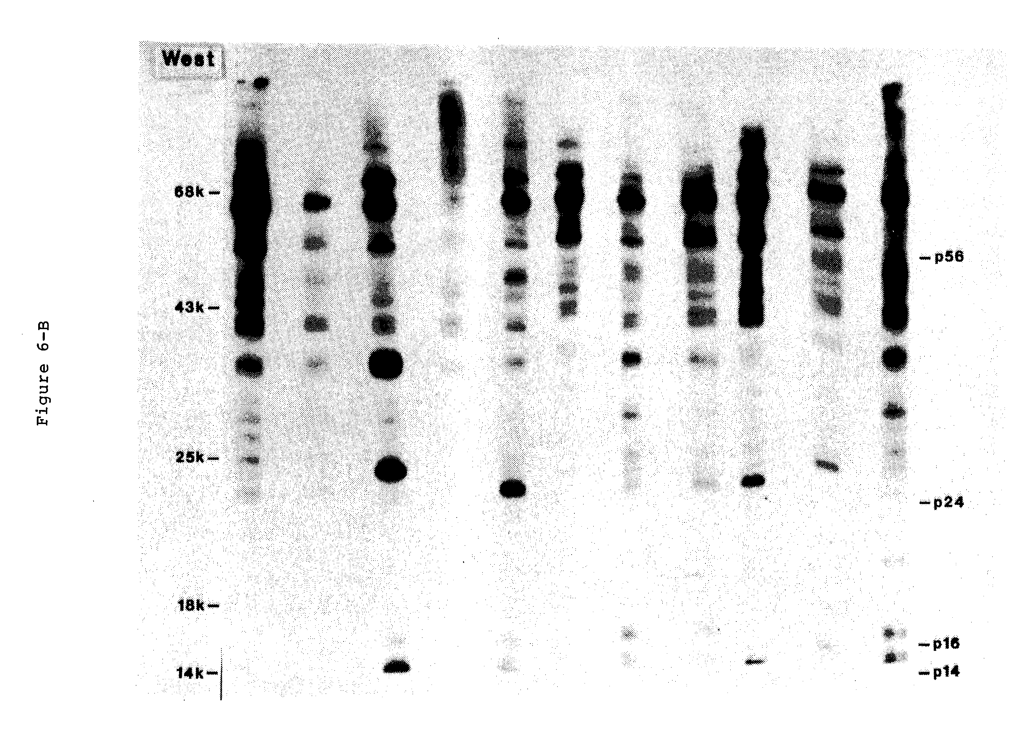

Also included in this invention are those amino acid substitution in the sequence of the 56, 48, 24, 16 and 14 kd gag proteins produced through mutation of the recombinant microorganisms of this invention. These amino acids substitutions in the sequence of these proteins produce modified proteins which are immunologically equivalent to the proteins of which they are modifications. These amino acid substitutions are described in the art - [H. Neurath and R.L. Hill "The Proteins", Academic Press, New York (1979)] in particular in fig. 6 of page 14. The most frequently observed amino acid substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly, and vice versa.

A further aspect of this invention relates to a diagnostic method for testing human blood for the presence of antibodies to the gag protein and its proteolytic proteins. This aspect of the invention overcomes the problems of previously used blood tests for AIDS. One of the problems in detecting in vitro the AIDS virus is to provide a composition which does not contain proteins or peptides which are not derived solely from the AIDS etiologic agent. A composition using either the active portion of gag-protein or its various proteolytically derived proteins overcomes the nonspecificity of the prior tests or assays. Yet another aspect of this invention is a diagnostic method for detecting and/or determining the presence of the antigen in human blood.

Another aspect of this invention is to use either the recombinant gag-protein or its proteolytically derived proteins as antigens in providing antibodies which are active in detecting AIDS in samples of body fluid.

Just another aspect of this invention is to use either the recombinant gag-protein or its proteolytically derived proteins as a vaccine capable of inducing protective immunity against the AIDS virus. Routes of administration, antigen doses, number and frequency of injections will vary from individual to individual and may parallel those currently being used in providing immunity in other viral infections. The vaccines can be prepared in accordance with known methods. The vaccine compositions will be conveniently combined with physiologically acceptable carrier materials. The vaccine compositions may contain adjuvants or any other enhancer of immune response. Furthermore, the vaccine compositions may comprise other antigens to provide immunity against other diseases in addition to AIDS.

The methods for testing human blood for the presence of AIDS virus or of antibodies against AIDS virus can be conducted in suitable test kits comprising in a container a recombinant gag-protein or its proteolytically derived proteins of the present invention or antibodies against AIDS virus elicited by these proteins of the present invention.

Brief Description of the Drawings.

Fig. I-A illustrates the restriction sites in λ HXB-3, a known gene clone for the HTLV-III virus, with the gag region of this gene expanded to show the precursor (p 56) and its naturally proteolytically produced proteins (p 24, p 16, and p 14) as well as its mutant protein (p 48).

Fig. I-B illustrates the construction of a gene containing the gag-gene for HTLV-III and a promoter for later insertion into a plasmid.

Fig. 2 is an immunoblot analysis of yeast lysates obtained from yeast grown with pYE72/gag I. Column I is the reading taken from cells grown in a high phosphate medium and column 2 is the reading from cells grown in a phosphate-free medium. The indicated molecular weight markers indicate the sizes of the respective bands.

Figure 3 is an autoradiograph reading of an SDS gel at various times after immunoprecipitated lysates produced from yeast cells containing the gag plasmid pYE72/gag I grown with $^{35}$S-methionine. Columns A through G in Fig. 3 represent different chase times. Column H represents results from lysates from yeast cells containing pYE 72/gag I grown with $^{32}$PO$_4$ and then harvested after 45 minutes and column I represents similar results as Column G except the plasmid inserted had no gag gene.

Fig 4 is an immunoblot analysis of yeast lysates produced through various mutations of the gag gene. The immunoblots were developed either with rabbit antibodies raised against disrupted HTLV-III (Part A of Fig. 4) or with AIDS patient serum (Part B of Fig. 4). In Parts A and B, column I is the result of lysates from cells induced with pYE72/gag I, while column 2 is the results of the lysates from cells induced with pYE72/gag 2 and column 3 is the results of the lysate from cells induced with pYE72/gag 3.

Fig 5 is the DNA sequence of that portion the λ HXB-3 gene showing the gag/pol overlap. The carboxy-terminal coding region of gag and the amino-terminal coding region of pol are shown. The region that is homologous to other gag proteases - [Toh et al., Nature 315, 691 (1985)] is underlined. The reading frame to which the pol gene is shifted by the BclI fill in is shown by the arrow. periods i.e. "." in this figure indicate a translation termination site.

Fig. 6-A is an immunoblot analysis utilizing antigen produced by the lysates of yeast cells induced with pYE72/gagI, each of the columns represents blood samples taken from a different AIDS patients living at the east coast of the United States.

Fig. 6-B is the same as Fig. 6-A except that the patients are taken from the west coast of the United States.

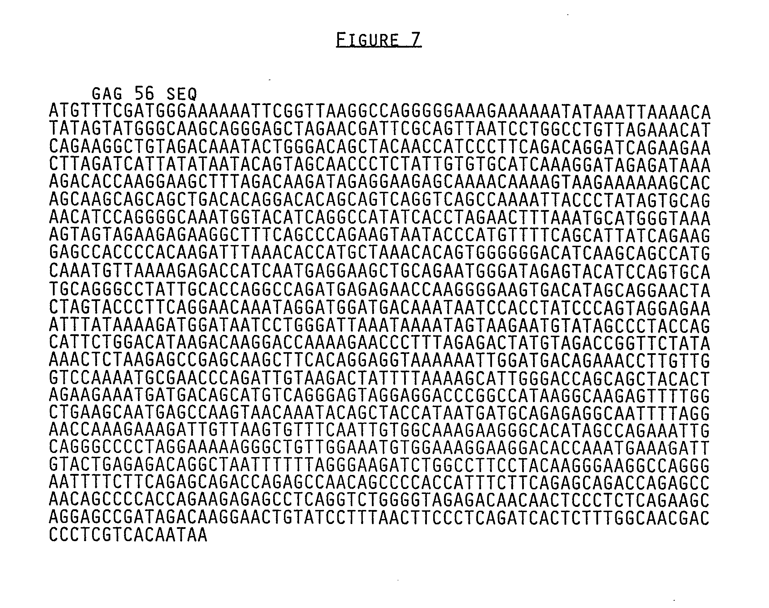

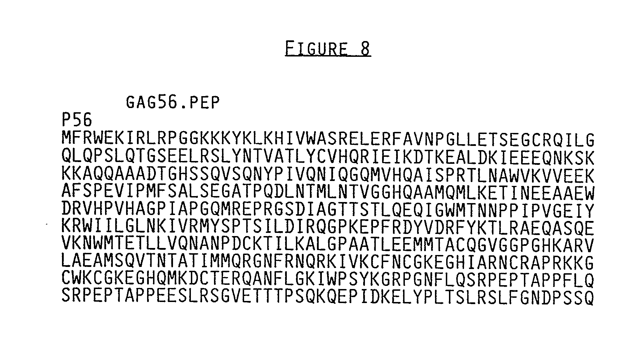

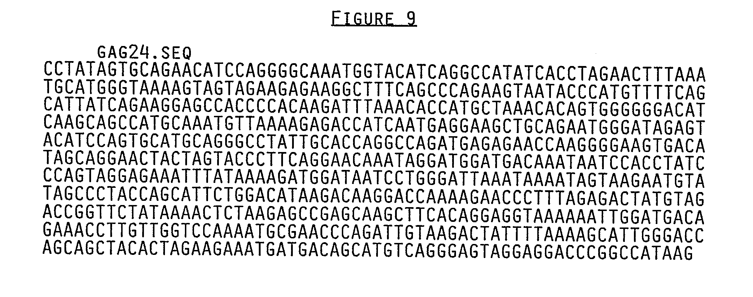

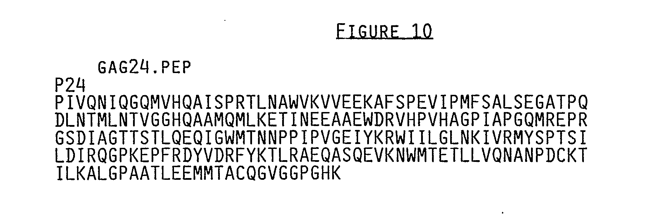

Fig. 7 is the DNA sequence encoding the 56 kd gag protein precusor produced in accordance with this invention.

Fig. 8 is the amino acid sequence of the 56 kd gag protein precusor produced in accordance with this invention.

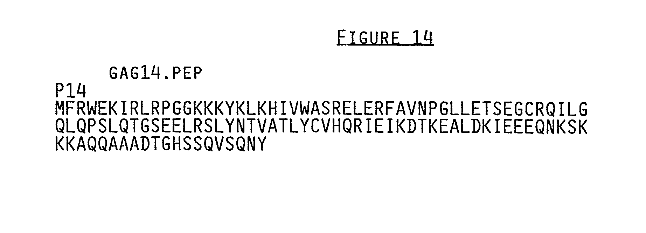

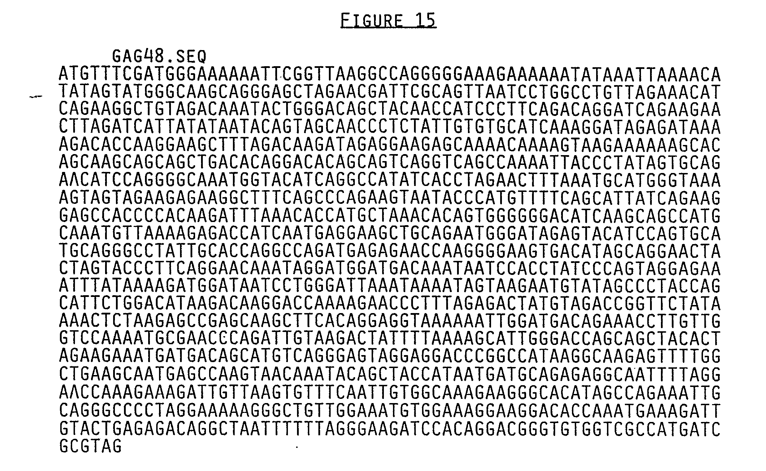

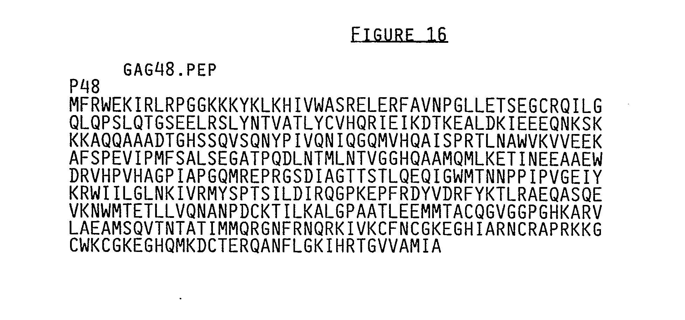

Fig. 9 is the DNA sequence encoding the proteolytic 24 kd gag protein produced in accordance with this invention.

Fig. l0 is the amino acid sequence of the 24 kd proteolytic gag protein is produced in accordance with this invention.

Fig. ll is the DNA sequence encoding the proteolytic l6 kd gag protein produced in accordance with this invention.

Fig. l2 is the amino acid sequence of the l6 kd proteolytic gag protein produced in accordance with this invention.

Fig. l3 is the DNA sequence encoding the l4 kd proteolytic gag protein produced in accordance with this invention.

Fig. l4 is the amino acid sequence of the l4 kd proteolytic gag protein produced in accordance with this invention.

Fig. l5 is the DNA sequence encoding the 48 kd proteolytic gag protein produced in accordance with this invention.

Fig. l6 is the amino acid sequence of the 48 kd proteolytic gag protein produced in accordance with this invention.


Detailed Description of the Invention


In the description the following terms are employed:

Nucleotide: A monomeric unit of DNA consisting of a sugar moiety (pentose), a phosphate, and either a purine or pyrimidine base (nitrogenous heterocyclic). The base is linked to the sugar moiety via the glycosidic carbon (l' carbon of the pentose). That combination of a base and a sugar is called a nucleotide. Each nucleotide is characterized by its base. The four DNA bases are adenine - ("A"), guanine ("G"), cytosine ("C") and thymine - ("T").

DNA Sequence: A linear array of nucleotides connected one to the other by phosphodiester bonds between the 3' and 5' carbons of adjacent pentoses.

Codon : A DNA sequence of three nucleotides (a triplet) which encodes through mRNA an amino acid, a translation start signal or a translation termination signal. For example, the nucleotide triplets TTA, TTG, CTT, CTC, CTA and CTG encode for the amino acid leucine ("Leu"). TAG, TAA and TGA are translation stop signals and ATG is a translation start signal.

Reading Frame: The grouping of codons during translation of mRNA into amino acid sequences. During translation the proper reading frame must be maintained. For example, the sequence GCTGGTTGTAAG may be translated in three reading frames or phases, each of which affords a different amino acid sequence:

GCT GGT TGT AAG--Ala-Gly-Cys-Lys
G CTG GTT GTA AG--Leu-Val-Val
GC TGG TTG TAA G--Trp-Leu-(STOP)

Polypeptide : A linear array of amino acids connected one to the other by peptide bonds between the α-amino and carboxy groups of adjacent amino acids.

Genome: The entire DNA of a cell or a virus. It includes inter alia the structural genes coding for the polypeptides of the substance, as well as operator, promoter and ribosome binding and interaction sequences, including sequences such as the Shine-Dalgarno sequences.

Structural Gene : A DNA sequence which encodes through its template or messenger RNA ("mRNA") a sequence of amino acids characteristic of a specific polypeptide.

Transcription: The process of producing mRNA from a structural gene.

Translation: The process of producing a polypeptide from mRNA.

Expression: The process undergone by a structural gene to produce a polypeptide. it is a combination of transciption and translation.

Plasmid : A circular double-stranded DNA Molecule that is not a part of the main chromosome of an organism containing genes that convey resistance to specific antibiotics. When the plasmid is placed within aunicellular organism, the characteristics of that organism may be changed or transformed as a result for the DNA of the plasmid. For example, a plasmid carrying the gene for tetracycline resistance (Tet^R) transforms a cell previously sensitive to tetracycline into one which is resistant to it. A cell transformed by a plasmid is called a "transformant."

Cloning Vehicle : A plasmid, phage DNA or other DNA sequences which are able to replicate in a host cell, which are characterized by one or a small number of endonuclease recognition sites at which such DNA sequences may be cut in a determinable fashion without attendant loss of an essential biological function of the DNA, e.g., replication, production of coat proteins or loss of promoter or binding sites, and which contain a marker suitable for use in the identification of transformed cells, e.g., tetracycline resistance or ampicillin resistance. A cloning vehicle is often called a vector.

Cloning: The process of obtaining a population of organisms or DNA sequences derived from one such organism or sequence by asexual reproduction.

Recombinant DNA Molecule or Hybrid DNA: A molecule consisting of segments of DNA from different genomes which have been joined end-to-end outside of living cells and have the capacity to infect some host cell and be maintained therein.

The nomenclature used to define the peptides or proteins is that used in accordance with conventional representation such that the amino group at the N-terminus appears to the left and the carboxyl group at the C-terminus to the right. By natural amino acid is meant one of common, naturally occurring amino acids found in proteins comprising Gly, Ala, Val, Leu, Ile, Ser, Thr, Lys, Arg, Asp, Asn, Glu, Gln, Cys, Met, Phe, Tyr, Pro, Trp and His. Where the amino acid residue has isomeric forms, it is the L-form of the amino acid that is represented unless otherwise expressly indicated. In addition, amino acids have been designated by specific letters of the alphabet such that: A = Alanine; D = Aspartic Acid; N = Asparagine; C = Cysteine; D = Aspartic Acid; E = Glutamic Acid; F = Phenylalanine; G = Glycine; H = Histidine; I = Isoleucine; K = Lysine; L = Leucine; M = Methionine; N = Asparagine; P = Proline; Q = Glutamine; R = Arginine; S = Serine; T = threonine; V = Valine; W = Tryptophan; Y = Tyrosine; Q = Glutamine; E = Glutamic Acid.

In accordance with the present invention, the search for the protein of the etiologic agent for acquired immune deficiency syndrome (AIDS) has led to the isolation and sequencing of the proviral gene of the AIDS virus. It has now been discovered, for what is believed to be the first time that the postulated etiologic agents of AIDS, lymphadenopathy-associated virus (LAV), AIDS-Associated retrovirus (ARV) and human T-cell leukemia/lymphoma/lymphotropic virus (HTLV III) are in fact variants of the same virus. For purposes of this invention and claims the virus causing AIDS will be referred to herein as HTLV-III virus. HTLV-III virus will be understood to include the variants which have been postulated as the causative agent of AIDS, namely LAV and ARV .

As seen in Fig. I, the genome for HTLV-III is known, i.e. λHXB-3 and contains regions which code for the gag-protein, pol protein, sor-protein and envelope(env)-protein. The region of the genome which codes for the gag-protein is found within the 5.5 kb EcoRI fragment region and more particular within the Cla I through Bcl I region.

In accordance with this invention, in order to obtain the proteins of this invention, the HTLV-III gene is cut with one or more restriction enzymes to obtain the fragment which contains the gene encoding the gag-protein. This fragment is then ligated with a promoter to form a gene containing the promoter operably linked to a DNA sequence coding for the gag-protein. It is through this linking that modification at the amino terminus of the protein produced therefrom are introduced when expressed in an organism. In the next step the gene containing the promoter and DNA sequence encoding the gag-protein of HTLV-III is then inserted into a plasmid or expression vector replicable in a suitable microbiological host to form a plasmid or expression vector containing the promoter operably linked to the DNA sequence encoding the gag-protein for HTLV-III.

In the current state of the art, there are a number of promoter systems and suitable microbial hosts available which are appropriate to the present invention. Also, there are many types of plasmids into which the gene encoding the gag-protein of HTLV-III can be inserted. In general, plasmid expression vectors containing replication and controlled sequence, which are derived from species compatable with the host cell are used in connection with these hosts. For example, E. coli is typically transformed using plasmid pBR322, a plasmid derived from an E. coli species. For use with yeast, such as S. cerevisiae a plasmid such as pYE7 is generally utilized.

In accordance with this invention, any conventional promoter compatible with the host and the plasmid selected can be utilized. Promoters used for recombinant DNA construction in E. coli include the beta-lactamase (penicillinase) and lactose promoter such as disclosed by Chang et al., Nature 275; 6I5 (1978); Itakura et al., Science, I98; I056 - (1977); promoter systems such as disclosed by Andersen et al., Mol. Cel. Bol. 3, 562-569 (1983) and Tryptophan promoter systems such as disclosed by Goeddel et al., Nucleic acids Res. 8, 4057 (1980) also EPO Appl. No. 0036776. In yeast, promoters including but being not limited to those from ADCI, GALI, GALI0, PHO5, PGKI and GAPI have been used as reviewed by Broach et al. - (1983) in Experimental Manipulation of Gene Expression, M. Inouye, ed., Academic press: New York, N.Y., pp 83-II7. While these are the most commonly used, other microbial promoters have been discovered and utilized and details concerning their nucleotide sequences have been published, enabling a skilled worker to ligate them functionally in an operable relationship to the genes in transformation vectors [Sibenlist et al., Cell 20, 269 (1980)].

A wide variety of host/cloning vehicle combinations may be employed in cloning the double-stranded DNA. For example, useful cloning vehicles may consist of segments of chromosomal, nonchromosomal and synthetic DNA sequences, such as various known bacterial plasmids, e.g., plasmids from E. coli such as pBR322, phage DNA, and vectors derived from combinations of plasmids and phage DNAs such as plasmids which have been modified to employ phage DNA or other expression control sequences or yeast plasmids. Useful hosts may include microorganisms, mammalian cells, plant cells and the like. Among them microorganisms and mammalian cells are preferably employed. As preferable microorganisms, there may be mentioned yeast such as S. cerevisiae and bacteria such as Escherichia coli, Bacillus subtillis, Bacillus stearothermophilus and Actinomyces. The above-mentioned vectors and hosts may also be employed for the production of a protein from a gene obtained biologically as in the instant invention. Of course, not all host/vector combinations may be equally efficient. The particular selection of host/cloning vehicle combination may be made by those skilled in the art after due consideration of the principles set forth without departing from the scope of this invention.

Furthermore, within each specific cloning vehicle, various sites may be selected for insertion of the double-stranded DNA. These sites are usually designated by the restriction endonuclease which cuts them. For example, in pBR322, the EcoRI site is located just outside the gene coding for ampicillin resistance. Various sites have been employed by others in their recombinant synthetic schemes. Several sites are well recognized by those of skill in the art. It is, of course, to be understood that a cloning vehicle useful in this invention need not have a restriction endonuclease site for insertion of the chosen DNA fragment. Instead, the vehicle could be joined to the fragment by alternative means.

The vector or cloning vehicle and in particular the site chosen therein for attachment of a selected DNA fragment to form a recombinant DNA molecule is determined by a variety of factors, e.g. number of sites susceptible to a particular restriction enzyme, size of the protein to be expressed, susceptibility of the desired protein to proteolytic degradation by host cell enzymes, contamination of the protein to be expressed by host cell proteins difficult to remove during purification, expression characteristics, such as the location of start and stop codons relative to the vector sequences, and other factors recognized by those of skill in the art. The choice of a vector and an insertion site for a particular gene is determined by a balance of these factors, not all selections being equally effective for a given case.

There are several known methods of inserting DNA sequences into cloning vehicles to form recombinant DNA molecules which are equally useful in this invention. These include, for example, direct ligation, synthetic linkers, exonuclease and polymerase-linked repair reactions followed by ligation, or extension of the DNA strand with DNA polymerase and an appropriate single stranded template followed by ligation.

It should, of course, be understood that the nucleotide sequences of the DNA fragment inserted at the selected site of the cloning vehicle may include nucleotides which are not part of the actual structural gene for the desired polypeptide/protein or may include only a fragment of the complete structural gene for the desired protein. It is only required that whatever DNA sequence is inserted, a transformed host will produce a protein/peptide having an immunological activity to the AIDS gag-protein or that the DNA sequence itself is of use as a hybridization probe to select clones which contain DNA sequences useful in the production of polypeptides/proteins having an immunological activity to the AIDS gag-protein.

The cloning vehicle or vector containing the foreign gene is employed to transform a host so as to permit that host to express the protein or portion thereof for which the hybrid DNA codes. The selection of an appropriate host is also controlled by a number of factors recognized by the art. These include, for example, compatibility with the chosen vector, toxicity of proteins encoded by the hybrid plasmid, ease of recovery of the desired protein, expression characteristics, biosafety and costs. A balance of these factors must be struck with the understanding that not all hosts may be equally effective for expression of a particular recombinant DNA molecule.

Once the organism capable of carrying out the expression of the gag gene has been created, the process of this invention can be carried out in a variety of ways depending upon the nature of the construction of the expression vectors for the gag gene and upon the growth characteristics of the host. Typically, the host organism will be grown under conditions which are favorable to production of a large quantities of cells. When a large number of cells has accumulated suitable inducers or derepressors in the growth medium cause the promoter supplied with such gene sequence to become active permitting the transcription and translation of the coding sequence. The protein produced by the recombinant cell can be lysated by conventional means well known in the art. It is apparent that the particular means of lysating will depend upon the host cell utilized.

In a preferred embodiment of this invention, the HTLV-III gag gene is introduced into a yeast expression vector with a promoter which is not activated in a phosphate medium. This promoter was obtained from PHO5 as described by Thill et al., Mol. Cell Biol. 3, 570-579 (1983). The gag gene was obtained from a 5.5Kb Eco RI fragment from the HTLV-III clone λ HXB-3 [Shaw et al. Science 226, II65-II7I (I984)]. This is the Eco RI fragment of the HTLV-III genome illustrated in Fig. I-A.

Fig. I-B illustrates the formation of the hybrid gene containing the PHO5 promoter and the gag gene fragment in Fig. I-A. The yeast promoter and translation initiation site are on a 560bp BamHI to Ahalll restriction fragment derived from the gene for repressible acid phosphatase, PHO5. The enzyme Ahalll produces a blunt end just after the second codon of PHO5 as seen in Fig. I-B. The gag gene within the 5.5 kd EcoRI fragment is ligated to the promoter obtained from the BamHI to Ahalll restriction fragment derived from PHO5. The EcoRI fragment from the HTLV-III clone contains the entire gag gene and a large part of the pol gene which overlaps the gag gene in a different reading frame (Ratner et al., supra; Sanchez-Pescador et al., supra; Wain-Hobson et al., supra; and Muesing et al., supra). The EcoRI fragment was cut with ClaI near the amino terminus of the gag gene and the resulting 5' overlap was filled in with DNA polymerase large fragment to create a blunt-ended DNA molecule beginning with an arginine codon. Ligation of this end to the Ahalll of the PHO5 fragment fused the promoter and the first two codons of PHO5 to the fifteenth codon of the gag gene.

The fused gene prepared above was then inserted into a pYE7 vector, a vector that can both replicate in yeast and E. coli. This recombinant plasmid was labelled pYE72/gagl. The resulting plasmid was then used to transform yeast.

In carrying out this ligation, any conventional method of ligation can be utilized to fuse the promoter to the gag gene. Any conventional method of transforming a microorganism such as yeast with a plasmid can be utilized to produce the recombinant organism which will express the gag gene.

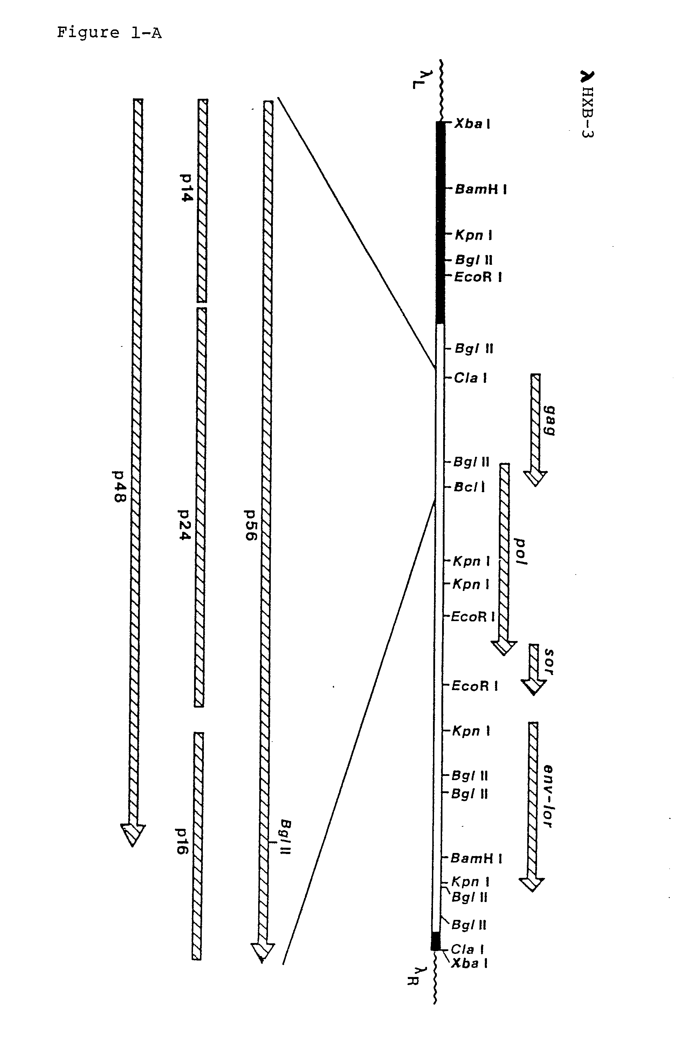

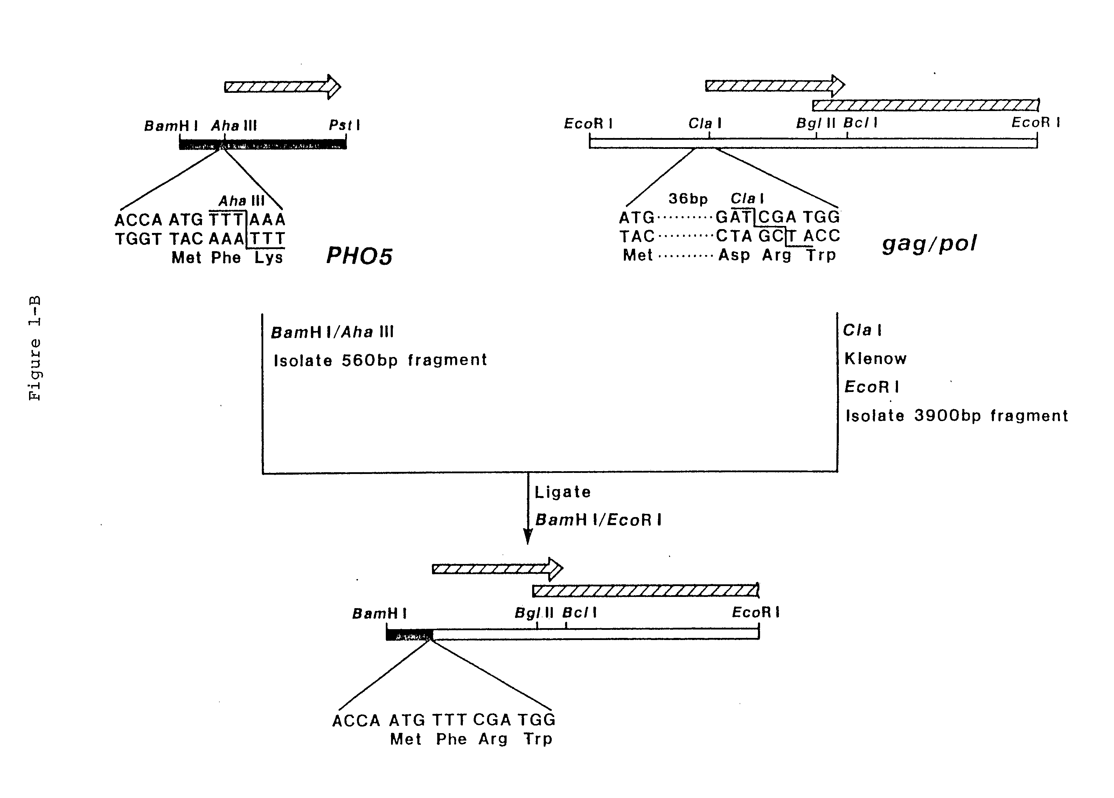

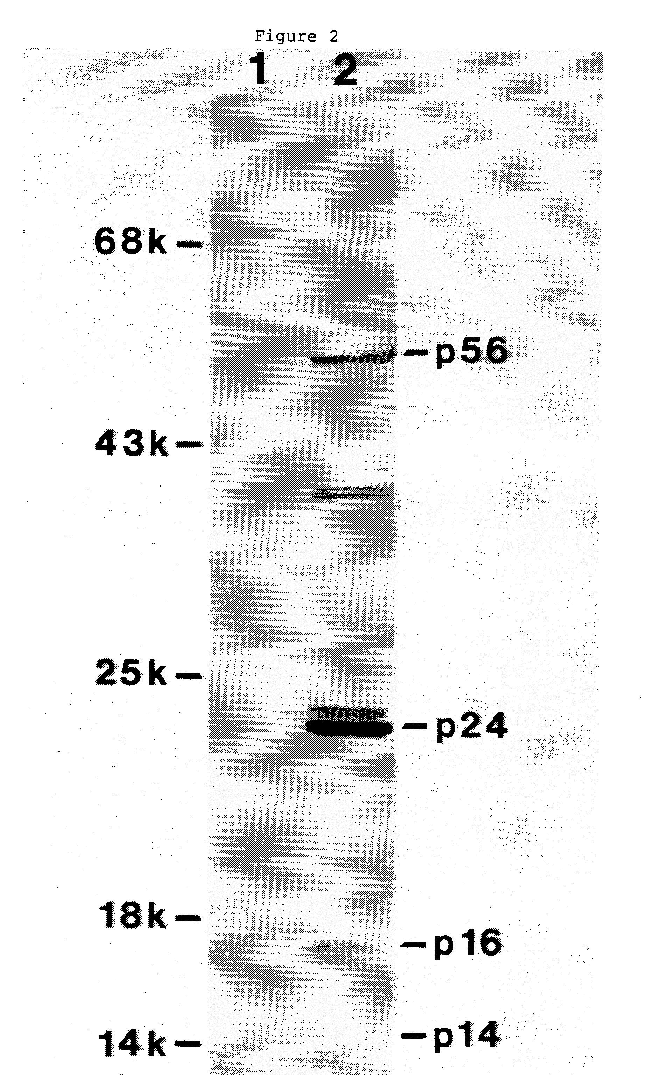

The PHO5 promoter in the plasmid labelled pYE72/gagl was induced in the yeast cells by growth in a phosphate-free medium and extracts of the cells were analyzed for the present of the gag-specific proteins by immunoblot analysis using rabbit polyclonal antiserum to disrupted virus as shown in Fig. 2. The column labelled I, in Fig. 2,

represents the results from the cells with pYE72/gagl grown in a high phosphate containing medium whereas column 2 represents the results from cells with pYE72/gagl grown in a phosphate-free medium. The indicated molecular weights are used to determine the sizes of the reactive band. As seen from Fig. 2, there was no expression of the gag gene in the phosphate containing medium were the promoter was not activated to produce the gag gene. On the other hand, when the same cells were grown in a phosphate-free medium, the lysates produced immuno reactive proteins which correspond to the gag protein and its various known proteolytic proteins which are formed therefrom.

As seen from column 2 in Fig. 2, a major immunoreactive protein produced corresponded in size to the HTLV-III p24 gag protein identified in virions and predicted from the known DNA sequence. The reactive species of the sizes expected for the pI4 and pI6 proteins were detected as well. A larger protein of about 56 kd size which corresponded to the entire gag protein as well as several species of about 40 kd which represents a proteolytic processing intermediate were also produced.

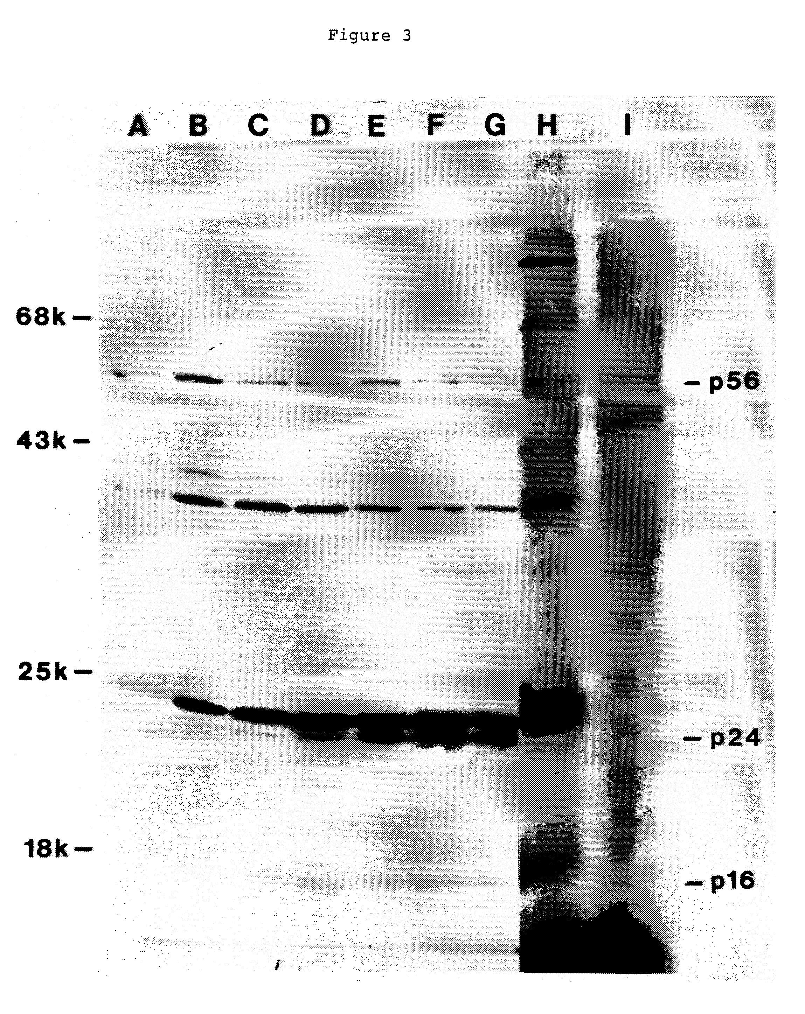

To determine whether the recombinant HTLV-III gag protein is actually processed by proteolysis in yeast to produce the actual viral specific proteolytic proteins, the proteins obtained from the lysate of the yeast transformed with pYE72/gagl was followed in a pulse chase experiment with $S^{35}$-methione or $^{32}PO_4$. Fig. 3 shows the results of this experiment, each of the columns represent a different chase time as follows: A) 0 min, B) 2 min, C) 5 min, D) I0 min, E) 20 min, F) 30 min, G) 45 min. In all respects the complete gag gene of 56 kd was detected with radioactivity first. The protein of about 25 kd seen in Fig. 2 was also detected before the 24 Kd protein and thus may be its immediate precursor. Protein migrating as expected of I6 Kd was also detected. The pI4 region of the gag gene has no methionine. Therefore, the failure to detect this protein supports its proposed origin.

Phosphorylation of the recombinant gag proteins made in yeast was examined after growth of the yeast cells transformed with pYE72/gagl in a low phosphate medium containing $^{32}PO_4$. In Fig. 3 columns H and I show the results of gel analysis of labelled proteins immunopercipitated with rabbit HTLV-III antibodies. In column H where the transformed yeast was utilized, the 56 kd, one of the 40 kd intermediates, the 25 kd, the 24 kd and the I6 kd species were all radioactive. No radioactivity was seen at the position of the I4 kd band when a higher percentage of gel was run. Thus the p24 and pl6 gag protein appear to be phosphorylated in yeast. No gag proteins were seen when the non-transformed yeast was utilized as in column I.

As seen from Fig. I-A presence of a BglII restriction site near the carboxy-terminal coding region of the gag gene provides a means to eliminate about half of the pl6 coding region as well as most of the pol gene present in 5.5 kd EcoRI fragment. The carboxy-terminal part of the gag polyprotein and/or the amino terminal region of the pol gene has been shown to encode a processing protease in many retroviruses (see, for example, Dickson et al., supra).

In order to remove the protease from the recombinant gag protein the BglII to EcoRI portion of the EcoRI fragment of HTLV-III genome was removed (see Fig. I-B). It is believed that the removed portion of the gag gene encodes proteases which convert the large recombinant gag protein into its various mature protein species. Therefore, the BamHI-EcoRI gene prepared in Fig. I-B can be fragmented by treating either the gene or the plasmid with BglII so as to remove the BglII to EcoRI fragment from this gene. If the expression plasmid pYE72/gagl is used, this plasmid is opened by digesting with BamHI and BglII. Once the BamHI to BglII portion of this EcoRI fragment is obtained, this portion may be inserted into a suitable plasmid, depending upon the microorganism into which it is to be grown. In inserting this portion of the gene into a plasmid it may be necessary to ligate this portion to series of DNA bases which permit insertion into the desired plasmid. In the case where pYE7 is utilized, the BamHI to BglII fragment is ligated with the 375 bp BamHI-EcoRI fragment from pBR322 to form a BamHI to EcoRI fragment which can be inserted into pYE7 to form pYE72/gag2.

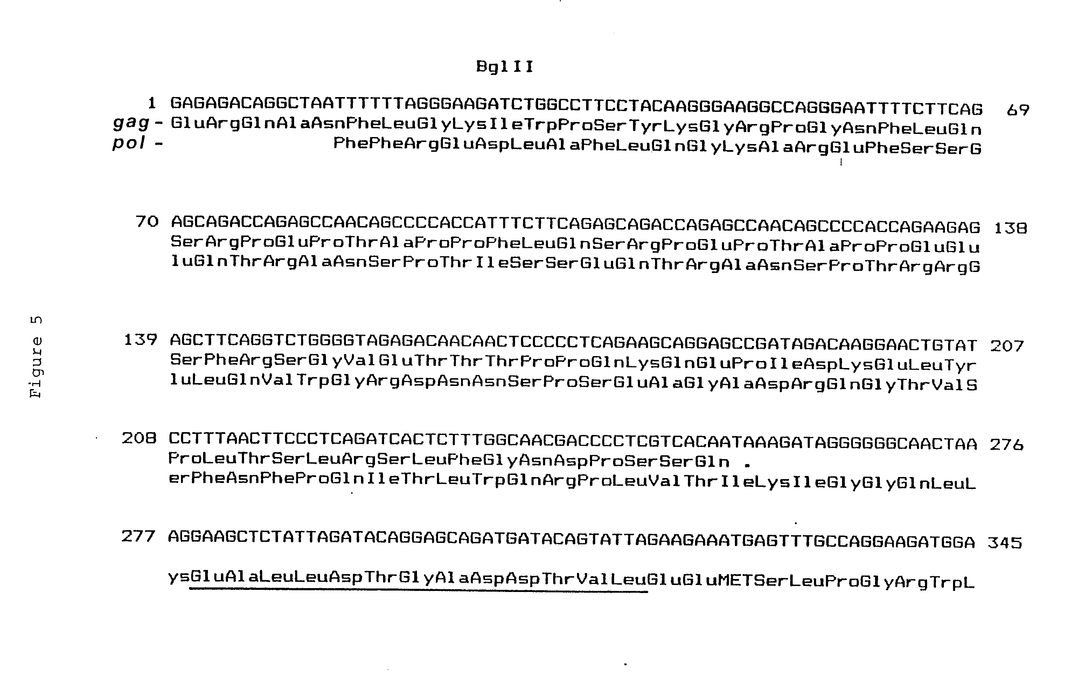

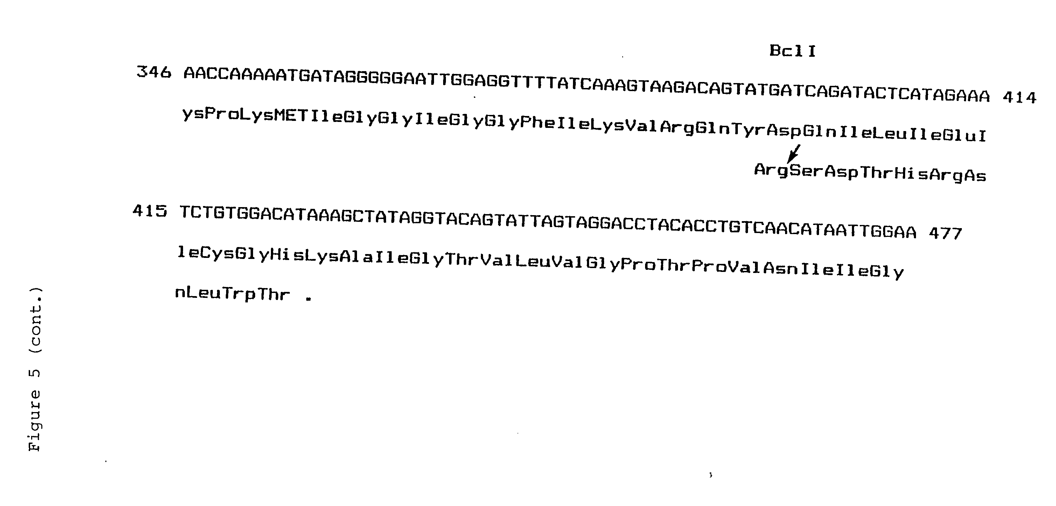

In accordance with another embodiment of this invention another mutation of the gag gene was introduced at the BclI restriction site in the pol gene just downstream of the gag, i.e. pYE72/gag3. This mutation involves introducing four base pairs at the BclI site which cause a frame shift out of the pol reading frame as shown in Fig. 5. In Fig. 5 the carboxy terminal coding region of the gag gene and the amino terminal coding region of the pol gene are shown. The region that is homologous to other gag proteases is underlined in this figure. The reading frame created by the BclI fill in is shown by the arrow. A period indicates a translational terminal site. The frame shift caused by the insertion of the bases deactivated the gene from producing certain proteases which may proteolytically cleave the gag protein.

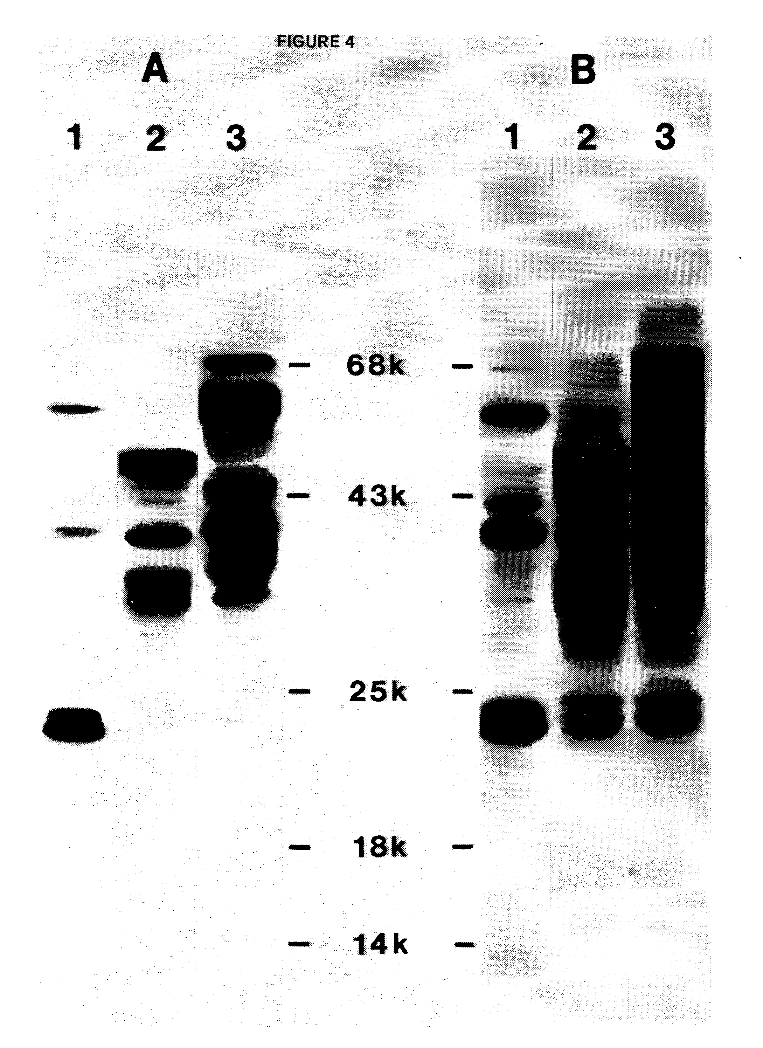

The products formed by the full recombinant gag gene and the truncated gag genes are shown in figure 4. Column I in Fig. 4 represent immunoblots taken from lysates of cells induced with pYE72/gagl. Column 2 in Fig. 4 is directed to the result of immunoblots taken from lysates from cells induced with pYE72/gag2; and column III represents immunoblots taken from lysates from cells induced with pYE72/gag3. As seen, pYE72/gagl and 3 produced the large protein p56. For pYE72/gag3, the major immunoreactive protein band was about 56 kd and comigrated with the presumptive precursor protein seen in the cells with induced pYE72/gagl. However, the mutation also appears to prevent processing of this recombinant gag precursor. As with the deletion mutant, a smaller amount of p24 appeared to be present as compared with protein seen in the cells induced with pYE72/gagl. In addition to a major 56 kd product and the smaller species, a band at about 60 kd was detected in the frame shift mutant. This size would be consistent with gag/pol fusion product that was terminated as shown in Fig. 5 as a result of the BclI filling in.

## Screening of AIDS SERA

Because anti-HTLV-III antibodies are found in more than 90% of the AIDS patients, the microbially synthesized gag gene products can be used as diagnostic tools for the detection of these antibodies. For this analysis as seen in Figures 6-A and 6-B, total cell protein from the yeast culture induced with pYE72/gagl was fractionated by SDS-PAGE and transferred to a nitrocellulose filter by Western blotting technique. Strips of the filter containing transferred proteins were reacted with I000-fold diluted human sera, and the antigen-antibody complexes formed were detected by incubation of the strips with I25-I-labelled Staphylococous aureus protein A followed by autoradiography. Prominent bands corresponding to reaction of the antibody to the 56 kd, 24 kd, I6 kd and I4 kd proteins were consistently observed when the serum used was from patients with AIDS syndrome. The results of one such assay with II human sera from patients on the East cost are presented in Figure 6-A. Similar results with II serum samples from west cost patients appear in Fig. 6-B. The negative controls (not shown) used were normal human sera. No reaction observed with sera from healthy individuals.

It appears, therefore, that the recombinant gag gene products can be used as diagnostic reagents for the detection of AIDS associated antibodies. The recombinant gag gene products of the instant invention encompasses a large portion of the protein molecule and contains both the conserved and divergent portions of the molecule. In spite of the divergence observed between HTLV-III and ARV-2 sequences the recombinant gag protein products of the instant invention synthesized by the bacteria react with AIDS patient sera derived from both geographical locations of the United States. One hundred percent (100%) of AIDS patient sera (22 individual samples, II derived from the East Coast of the United States and II derived from California) tested showed high reactivity. This is strong evidence for the presence of conserved epitopes within the molecule against which the immune system could mount an antibody reaction. The human immune system may thus be mounting an immune response against conserved epitopes of the gag proteins molecule by the reactivity of the AIDS patient sera.

Based on these discoveries it is proposed that in the practice of screening blood for Acquired Immune Deficiency Syndrome (AIDS), the AIDS recombinant gag protein products of this invention can be utilized. Utilizing the protein products of the instant invention, human blood can be screened for the presence of antibodies to the AIDS virus. This and other techniques are readily determined. The foregoing and other objects, features and advantages of the invention will be apparent from the following examples of preferred embodiments of the invention.

In the Examples, E. coli strain MCI06I was the same as described by Casadaban et al., J. Mol. Biol. 138, 179-207 (1980). The E. coli strain GMII9 used for the preparation of the unmethylated DNA, was that described by Arraj et al., J. Bact. 153, 562-563 (1983). E. coli strains MC 106I and GM II9 were deposited at American Type Culture Collection - (ATCC) on November 26, 1985 the accession nos. being ATCC 53338 and ATCC 53339, respectively. The yeast expression plasmid pYE7 used in Example 5 was the same as described in Examples and Fig. 6 of European Patent Application publication No. 0124824 which is incorporated by reference. The yeast used was S. cerevisiae 20B-I2 (ATCC No. 20626). Yeast transformation was performed as described by Hinnen et al., Proc. Nat. Acad. Sci.. USA, 75, 1929-1934 (1978). The PHO5 gene utilized to produce the promoter was obtained from the plasmid pAP20 as described by Andersen et al., Mol. Cell Biol. 3 562-569 (1983). The λ HXB-3 used, was as described by Shaw et al., Science 226, 1165-1171 (1984).

## Example I

## Preparation of pYE72/gagI

Restriction and DNA modifying enzymes were used as recommended by the supplier. All restriction enzyme digests were performed at 37°C for I hr with 0.5-1.0 units of enzyme in 50mM NaCl, 10mM Tris-HCl, pH 7.4, 10mM MgCl₂, ImM dithiothreitol (DTT). The 560 bp BamHI to AhaIII fragment with PHO5 promoter and translation initiation region was obtained from the plasmid pAP20, and the ClaI to EcoRI fragment with the gag/pol region was obtained from λ HXB-3. The 5.5 kb. EcoI fragment was subcloned into pBR3222 and the plasmid grown in E. coli GMII9. The ClaI 5' overhang of the ClaI to EcoRI-fragment was "filled in" by treatment with 5 units of the Klenow fragment of E. coli DNA polymerase I in the presence of all four deoxyribonucleotides at 50μM (C,T,A, and G) at I6°C for 2 hr. in the same buffer as that used for restriction enzyme reactions.

Approximately equal amounts of the BamHI-AhaIII PHO5 fragment and the ClaI (filled-in)-EcoRI gag/pol fragment were treated with I.0 units of T4 DNA ligase for I6 hr at I6°C in 50mM Tris-HCl pH 7.8, 10mM MgCl₂, 20mM DTT, ImM αATP. The products were cut with BamHI and EcoRI and the PHO5-gag/pol fusion was inserted by ligation into pYE7 which had been cut with BamHI and EcoRI. The resulting expression plasmid was designated pYE72/gagI.

## Example 2

## Preparation of pYE72/gag2

A deletion that removed the carboxy-terminal portion of gag and all of pol was made by digesting pYE72/gagI with BamHI and BglII and isolating the PHO5-gag fragment. This was converted to a BamHI to EcoRI fragment by ligation with the 375 bp BamHI-EcoRI fragment from pBR322 to the BglII site through the identical 5' overhangs of BglII and BamHI. Insertion of this fragment into pYE7 yielded PYE72/gag2.

## Example 3

## Preparation of pYE72/gag3

Another mutation was introduced at the BclI site in the pol gene just downstream of gag. Since BclI does not cut methylated DNA, pYE72/gagI was introduced into E. coli GMII9, and DNA was prepared and cut with BclI. The 5' overhang was filled in with Klenow fragment as above and the plasmid was recircularized by blunt-end ligation. The resulting plasmid with the 4 bp GATC insert at the BclI site was designated pYE72/gag3.

## Example 4

### Growth and induction of Yeast Strains

Separate cultures of the yeast strain S. cerevisiae 20B-I2 containing the gag expression plasmids or a vector with no inserted gene were grown in the medium YCAD and induced in phosphate-free medium as described by Kramer et al., Proc. Natl. Acad. Sci USA 8I, 367-370 (I984). About 6 hr after transfer to phosphate-free medium, cells were collected by centrifugation and spheroplasts prepared with Zymolyase 60,000 - [Kaneko, T., Kitamura, K., and Yamamoto, Y., Agr. Biol. Chem., 37, 2295 (I973)] and collected. spheroplasts were usually stored at -20°C before analysis. For protein purification, whole cells instead of spheroplasts are collected and frozen.

## Example 5

### Purification of the recombinant gag Protein Products

A homogeneous recombinant gag protein or its proteolytic products can be purified according to the following procedure. The induced yeast cells are broken by standard mechanical procedures. These include passage through a French Pressure Cell or rapid mixing with glass beads in a mixer such as a Bead Beater, a Dyna-Mill or a Braun Homogenizer. For any of the above, the cell paste is resuspended in approximately 2 volumes - (relative to cell pellet) of 50mM $NaPO_4$, pH7.4 buffer. For glass bead lysis, an equal volume of 0.5 mm glass beads is added. Lysis is accomplished by either three passages at 20,000 p.s.i. through the French Pressure Cell or as recommended by the manufacturer of the mixer. Lysis can be monitored by microscopy.

Following cell breakage, cell debris (and glass beads) are removed by two I0 min centrifugation at $600^x g$. Another centrifugation at I2,000$^x$g for 20 min removes mitochondria. The proteins are then fractionated by centrifugation at I00,000$^x$g for I hr to obtain a pellet (microsomal fraction) and supernatant (soluble fraction). If the gag proteins are in the microsomal fraction, solubilization using either various ionic and/or non-ionic detergents or denaturing reagents such as urea or guanidine HCl is necessary prior to further purification.

Additional purification can be achieved by standard liquid chromatography procedures. Different chromatography media can be used to obtain fractionation by gel filtration, ion exchange chromatography, and/or affinity chromatography. For gag proteins, single-stranded DNA cellulose affinity chromatography should be useful since gag proteins bind to nucleic acids.

Final purification can be obtained by reverse phase high performance liquid chromatography - (HPLC). The HPLC step yields the precursor gag protein and the natural proteolysis proteins derived therefrom in a substantially I00% pure form. It is also foreseeable that monoclonal antibody affinity chromatography columns utilizing gag polyclonal or monoclonal antibodies to the precursor gag protein and the natural proteolysis proteins, could be used as an alternative to HPLC.

By the above purification procedure, one obtains the following products:

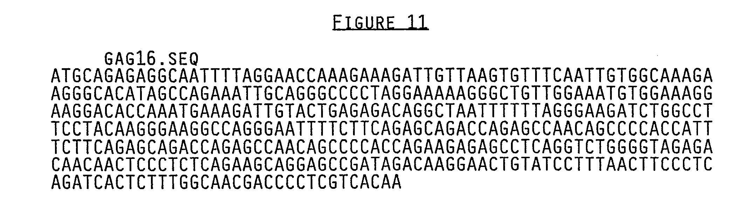

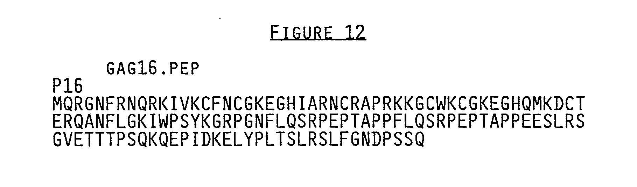

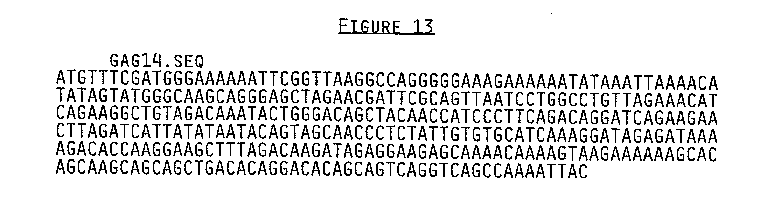

The 56 kd protein having the structure given in Fig 8;

The 24 kd protein having the structure given in Fig I0;

The I6 kd protein having the structure given in Fig I2;

The I4 kd protein having the structure given in Fig I4;

and

The 48 kd protein having the structure given in Fig I6.

## Example 6

### Polyacrylamide Gel Electrophoresis and Western Blot Analysis

For the immunoblot analysis of Figs. 2, 3, 4, 6-A and 6-B, cells were lysed by resuspending the spheroplast pellets (approximately $10^8$ cells) in an equal volume of 2 $^x$ sample buffer of Laemmli - (Laemmli, "Cleavage of Structural Proteins During the Assembly of the Head of Bacteriophage T4", Nature 227, 68-685, I970) and incubated at 95°C for five (5) minutes. Debris were pelleted by centrifugation and the cleared lysates were subjected to SDS-PAGE analysis, Id. For Western blot analysis, the proteins from the acrylamide gel were electroblotted onto a 0.I micrometers nitrocellulose membrane (Schleicher and Schuell) for I6 hr at 50V, in I2.5 mM Tris, 96 mM glycine, 20% methanol, 0.0I% SDS at pH 7.5. Processing of the blot was carried out using the methods described by Towbin et al., "Electrophoretic Transfer of Proteins From Polyacrylamide Gels to Nitrocellulose Sheet: Procedure and Some applications," Proc. Natl. Acad. Sci. U.S.A., 76, 4350-4354, (I979). For treat-

ment with the human sera, the blots were incubated with a I000 fold dilution of the sera in antibody buffer (20 mM sodium phosphate buffer, pH 7.5 containing 0.5 M NaCl, I% BSA and 0.05% Tween 20) for 2-6 hr. The blots were then washed twice with phosphate buffered saline containing 0.05% Tween 20 and then incubated with I25-I-labelled Staphylococous aureus protein A for an additional period of I hr. The blot was then washed twice in PBS-Tween 20 buffer, dried and autoradiographed.

## Example 7

## Labelling of the Cells

The cells were labelled with either $^{35}$S-methionine pulse-chase or $^{32}$PO$_4$ for the pulse chase immunopre cipitation of Fig. 3 by the following procedure. For the $^{35}$S-methionine pulse-chase, 500 mCi $^{35}$S-methionine was added to the culture medium. After a 2 min labelling period, unlabelled methionine was added. At various times, the cells were harvested, lysed and processed by immunoprecipitation with rabbit antibody, and the lysates were analyzed by SDS-PAGE and autoradiography. The chase times are as follows: A) 0 min, B) 2 min, C) 5 min, D) I0 min, E) 20 min, F) 30 min, G) 45 min. For the $^{35}$PO$_4$ labeling in Columns H and I, I mCi $^{32}$PO$_4$ was added to the culture medium. Labeling was continued for 30 min. at 30°C, then cells were harvested, and lysates processed for immunoprecipitation as described above. Lysates were from cells containing pYE72/gagI (H), and cells containing a similar plasmid with no gag gene (I).

## Example 8

## Diagnostic Test for AIDS

It is clear that the recombinant precursor gag protein and the natural proteolysis proteins derived therefrom, of the instant invention may be used as diagnostic reagents for the detection of AIDS-Associated antibodies. It is also apparent to one of ordinary skill that a diagnostic assay for AIDS using polyclonal or monoclonal antibodies to the AIDS recombinant gag precursor protein or the prot-· eolytic products may be used to detect the presence of the AIDS virus in human blood. In one embodiment a competition immunoassay is used where the antigenic substance, in this case the AIDS virus, in a blood sample competes with a known quantity of labelled antigen, in this case labelled AIDS recombinant precursor gag protein, or the proteolysis proteins derived therefrom for a limited quantity of antibody binding sites. Thus, the amount of labelled antigen bound to the antibody is inversely proportional to the amount of antigen in the sample. In another embodiment, an immunometric assay may be used wherein a labelled AIDS gag antibody which complexes with the antigen-bound antibody is directly proportional to the amount of antigen (AIDS virus) in the blood sample. In a simple yes/no assay to determine whether the AIDS virus is present in blood, the solid support is tested to detect the presence of labelled antibody. In another embodiment, monoclonal antibodies to recombinant precursor AIDS gag protein, or the natural proteolysis proteins derived therefrom, may be used in an immunometric assay. Such monoclonal antibodies may be obtained by methods well known in the art, particularly the process of Milstein and Kohler reported in Nature 256, 495-497 (I975). The antigens in this assay can be the recombinant gag precursor protein or the proteolysis proteins derived therein either in pure form or as a mixture of these proteins.

The immunometric assay method is as follows: Duplicate samples are run in which I00 µl of a suspension of antibody immobilized on agarose particles is mixed with I00 µl of serum and I00 µl of soluble $^{125}$I-labelled antibody. This mixture is for specified times ranging from one-quarter hour to twenty-four hours. Following the incubation periods the agarose particles are washed by addition of buffer and then centrifuged. After removal of the washing liquid by aspiration, the resulting pellet of agarose particles is then counted for bound $^{125}$I-labelled antibody. The counts obtained for each of the complexes can then be compared to controls.

Various features of the invention are set forth in the following claims.

## Claims

I. A polypeptide immunologically equivalent to the gag-protein products of HTLV-III having the amino acid sequence given in either Fig. 8 or Fig. I6, or I4kd, I6kd or 24kd proteolytic polypeptide fragments thereof or polypeptides related to any of said polypeptides by amino acid substitution(s) which occur through mutations of a recombinant host cell which produces said polypeptides.

2. The polypeptide of claim I wherein said polypeptide is the 56kd precursor having the amino acid sequence:

MFRWEKIRLRPGGKKKYKLKHIVWASRELERFAV-NPGLLETSEGCRQILG QLQPSLQTGSEELRSLYN-TVATLVCVHQRIEIKDTKEALDKIEEEQNKSK KKAQQAAADTGHSSQVSQNYPIVQNIQGQMVHQA-ISPRTLNAWVKVVEEK AFSPEVIPMFSALSEGATP-

QDLNTMLNTVGGHQAAMQMLKETINEEAAEW
DRVHPVHAGPIAPGQMREPRGSDIAGTTSTLQEQI-
GWMTNNPPIPVGEIY KRWIILGLNKIVRMYSPTSIL-
DIRQGPKEPFRDYVDRFYKTLRAEQASQE
VKNWMTETLLVQNANPDCKTILKALGPAATLEEN-
MTACQGVGGPGHKARV LAEAMSQVTNTATIMM-
QRGNFRNQRKIVKCFNCGKEGHIARNCRAPRKKG
CWKCGKEGHQMKDCTERQANFLGKIWPSYKGRP-
GNFLQSRPEPTAPPFLQ SRPEPTAPPEESLRSG-
VETTTPSQKQEPIDKELYPLTSLRSLFGNDPSSQ

3. The polypeptide of claim I wherein the polypeptide is a I4kd proteolytic fragment having the amino acid sequence:
MFRWEKIRLRPGGKKKYKLKHIVWASRELERFAV-
NPGLLETSEGCRQILG QLQPSLQTGSEELRSLYN-
TVATLYCVHQRIEIKDTKEALDKIEEEQNKSK
KKAQQAAADTGHSSQVSQNY

4. The polypeptide of claim I wherein said polypeptide is the 48kd proteolytic fragment having the amino acid sequence:
MFRWEKIRLRPGGKKKYKLKHIVWASRELERFAV-
NPGLLETSEGCRQILG QLQPSLQTGSEELRSLYN-
TVATLYCVHQRIEIKDTKEALDKIEEEQNKSK
KKAQQAAADTGHSSQVSQNYPIVQNIQGGQMVHQA-
ISPRTLNAWVKVVEEK AFSPEVIPMFSALSEGATP-
QDLNTMLNTVGGHQAAMQMLKETINEEAAEW
DRVHPVHAGPIAPGQMREPRGSDIAGTTSTLQEQI-
GWMTNNPPIPVGEIY KRWIILGLNKIVRMYSPTSIL-
DIRQGPKEPFRDYVDRFYKTLRAEQASQE
VKNWMTETLLVQNANPDCKTILKALGPAATLEEM-
MTACQGVGGPGHKARV LAEAMSQVTNTATIMM-
QRGNFRNQRKIVKCFNCGKEGHIARNCRAPRKKG
CWKCGKEGHQMKDCTERQANFLGKIHRTGVVAM-
IA

5. The polypeptide of claim I wherein said polypeptide is the I6kd proteolytic fragment having the amino acid sequence:
MQRGNFRNQRKIVKCFNCGKEGHIARNCRAPRKK-
GCWKCGKEGHQMKDCT ERQANFLGKIWPSYKG-
RPGNFLQSRPEPTAPPFLQSRPEPTAPPEESLRS
GVETTTPSQKQEPIDKELYPLTSLRSLFGNDPSSQ

6. The polypeptide of claim I wherein said polypeptide is the 24kd proteolytic fragment having the amino acid sequence:
PIVQNIQGGQMVHQAISPRTLNAWVKVVEEKAFSPE-
VIPMFSALSEGATPQ DLNTMLNTVGGHQAAMQM-
LKETINEEAAEWDRVHPVHAGPIAPGQMREPR
GSDIAGTTSTLQEQIGWMTNNPPIPVGEIYKRWIIL-
GLNKIVRMYSPTSI LDIRQGPKEPFRDYVDRFYKT-
LRAEQASQEVKNWMTETLLVQNANPDCKT ILKAL-
GPAATLEEMMTACQGVGGPGHK

7. A polypeptide immunologically equivalent to the gag-protein products of HTLV-III expressed in yeast.

8. A gene containing a gene portion having a DNA sequence encoding a polypeptide as claimed in any one of claims I to 6 operably linked to a promoter capable of effecting the expression of said DNA sequence.

9. The gene of claim 8 wherein said gene portion is the ClaI to EcoRI restriction site fragment of the λ HXB-3 genome.

I0. The gene of claim 9 wherein the promoter is the BamHI to AhaIII restriction site fragment of PHO5.

II. The gene of claim 8 wherein said gene portion is the ClaI to BglII restriction site fragment of the ClaI to EcoRI fragment of the λ HXB-3 genome.

I2. The gene of claim II wherein the promoter is the BamHI to AhaIII restriction site fragment of PHO5.

I3. The gene of claim 8 where the gene portion is the ClaI to EcoRI restriction site fragment of λ HXB-3, wherein the ClaI to EcoRI fragment is filled in at its BclI restriction site with the base sequence GATC.

I4. The gene of claim I3 wherein the promoter is the BamHI to AhaIII restriction site fragment of PHO5.

I5. A gene containing a gene portion having a DNA sequence encoding a polypeptide immunologically equivalent to the gag-protein products of HTLV-III operably linked to a promoter capable of effecting the expression of said DNA sequence in yeast.

I6. A recombinant expression vector capable of effecting the expression of a polypeptide as claimed in any one of claims I to 6 containing a gene portion having a DNA sequence encoding said polypeptide operably linked to a promoter capable of effecting the expression of said DNA sequence.

I7. A vector according to claim I6 wherein the gene portion is the ClaI to EcoRI restriction fragment of the λ HXB-3 genome.

I8. A vector according to claim I7 wherein the promoter is the BamHI to AhaIII restriction site fragment of PHO5.

I9. A vector according to claim I6 wherein the gene portion is the ClaI to BglII restriction site fragment of the Cla I to EcoRI restriction site fragment of the λ HXB-3 genome.

20. A vector according to claim I9 wherein the promoter is the BamHI to AhaIII restriction site fragment of PHO5.

2I. A vector according to claim I6 wherein the gene portion is the ClaI to EcoRI restriction site fragment of λ HXB-3, wherein the ClaI to EcoRI fragment is filled in at the BclI restriction site with the base sequence GATC.

22. A vector according to claim 2l wherein the promoter is the BamHl to Ahalll restriction site fragment of PHO5.

23. A vector according to claim l8 which is pYE72/gagl.

24. A vector according to claim 20 which is pYE72/gag2.

25. A vector according to claim 22 which is pYE72/gag3.

26. A recombinant expression vector capable of effecting the expression of a polypeptide immunologically equivalent to the gag-protein products of HTLV-III in yeast containing a gene portion having a DNA sequence encoding the gag-protein of HTLV-III operably linked to a promoter capable of effecting the expression of said DNA sequence.

27. A transformed cell carrying a vector as claimed in any one of claims l6 to 25.

28. A transformed cell according to claim 27 which is a yeast cell.

29. A transformed cell according to claim 28 which is a S. cerevisiae yeast cell.

30. A transformed cell according to claim 29 which is S. cerevisiae 20B-l2.

3l. A transformed yeast cell carrying a vector as claimed in claim 26.

32. A polypeptide according to any one of claims l to 7 as constituent of a vaccine.

33. A polypeptide according to any one of claims l to 7 as antigen.

34. A process for producing a polypeptide as claimed in any one of claims l to 6 comprising: transforming a host cell with an expression vector as claimed in any one of claims l6 to 25; culturing said host cell so that the protein products are expressed; and, extracting and isolating said protein products.

35. A process according to claim 34 wherein said host cell is a yeast cell

36. A process according to claim 35 wherein said yeast cell is a S. cerevisiae cell.

37. A process for producing a polypeptide immunologically equivalent to the gag-protein products of HTLV-III comprising: transforming a yeast cell with an expression vector as claimed in claim 26; culturing said yeast cell so that the protein products are expressed; and, extracting and isolating said protein products.

38. A method of testing human blood for the presence of antibodies to the viral etiologic agent of AIDS which comprises mixing a composition containing a polypeptide as claimed in any one of claims l to 7 or mixtures thereof with a sample of human blood and determining whether said protein or any of its natural proteolytic proteins or mixtures thereof binds to AIDS antibodies present in the blood sample.

39. Vaccines containing a polypeptide as claimed in any one of claims l to 7 and a physiologically acceptable carrier.

40. Antibodies raised aqainst a polypeptid as claimed in any one of claims l to 7.

4l. The antibodies of claim 40 which are monoclonal antibodies.

42. The use of a polypeptide as claimed in any one of claims l to 7 for the preparation of a protective immunization vaccine.

43. The use of a polypeptide as claimed in any one of claims l to 7 for the preparation of antibodies against AIDS virus.

44. The use of a polypeptide as claimed in any one of claims l to 7 for testing human blood for the presence of AIDS virus.

45. A test kit for the determination of antibodies against AIDS virus comprising in a container a polypeptide according to any one of claims l to 7.

46. A test kit for the determination of AIDS virus comprising in a container antibodies against AIDS virus elicited by a polypeptide according to any one of claims l to 7.

Claims for the following Contracting States : AT; ES

l. A process for producing polypeptides immunologically equivalent to the gag-protein products of HTLV-III having the amino acid sequence given in Fig. l6 or Fig. l6, or l4kd, l6kd or 24kd proteolytic fragments thereof, which process comprises: transforming a host cell with an expression vector comprising a gene coding for said gag-protein products operably linked to a promoter sequence enabling transcription, translation and expression of said gag-protein products in said host cell; culturing said host cell so that the protein products are expressed; and, extracting and isolating said protein products.

2. A process according to claim l, characterized in that a host cell is transformed with an expression vector capable of expressing a polypeptide with the amino acid sequence: MFRWEKIRLRPGGKKKYKLKHIVWASRELERFAV-NPGLLETSEGCRQILG QLQPSLQTGSEELRSLYN-TVATLYCVHQRIEIKDTKEALDKIEEEQNKSK KKAQQAAADTGHSSQVSQNYPIVQNIQGQMVHQA-ISPRTLNAWVKVVEEK AFSPEVIPMFSALSEGATP-QDLNTMLNTVGGHQAAMQMLKETINEEAAEW DRVHPVHAGPIAPGQMREPRGSDIAGTTSTLQEQI-GWMTNNPPIPVGEIY KRWIILGLNKIVRMYSPTSIL-DIRQGPKEPFRDYVDRFYKTLRAEQASQE VKNWMTETLLVQNANPDCKTILKALGPAATLEEM-MTACQGVGGPGHKARV LAEAMSQVTNTATIMM-QRGNFRNQRKIVKCFNCGKEGHIARNCRAPRKKG

CWKCGKEGHQMKDCTERQANFLGKIWPSYKGRP-GNFLQSRPEPTAPPFLQ SRPEPTAPPEESLRSG-VETTTPSQKQEPIDKELYPLTSLRSLFGNDPSSQ

3. A process according to claim I, characterized in that a host cell is transformed with an expression vector capable of expressing a polypeptide with the amino acid sequence: MFRWEKIRLRPGGKKKYKLKHIVWASRELERFAV-NPGLLETSEGCRQILG QLQPSLQTGSEELRSLYN-TVATLYCVHQRIEIKDTKEALDKIEEEQNKSK KKAQQAAADTGHSSQVSQNY

4. A process according to claim I, characterized in that a host cell is transformed with an expression vector capable of expressing a polypeptide with the amino acid sequence: MFRWEKIRLRPGGKKKYKLKHIVWASRELERFAV-NPGLLETSEGCRQILG QLQPSLQTGSEELRSLYN-TVATLYCVHQRIEIKDTKEALDKIEEEQNKSK KKAQQAAADTGHSSQVSQNYPIVQNIQGQMVHQA-ISPRTLNAWVKVVEEK AFSPEVIPMFSALSEGATP-QDLNTMLNTVGGHQAAMQMLKETINEEAAEW DRVHPVHAGPIAPGQMREPRGSDIAGTTSTLQEQI-GWMTNNPPIPVGEIY KRWIILGLNKIVRMYSPTSIL-DIRQGPKEPFRDYVDRFYKTLRAEQASQE VKNWMTETLLVQNANPDCKTILKALGPAATLEEM-MTACQGVGGPGHKARV LAEAMSQVTNTATIMM-QRGNFRNQRKIVKCFNCGKEGHIARNCRAPRKKG CWKCGKEGHQMKDCTERQANFLGKIHRTGVVAM-IA

5. A process according to claim I, characterized in that a host cell is transformed with an expression vector capable of expressing a polypeptide with the amino acid sequence: MQRGNFRNQRKIVKCFNCGKEGHIARNCRAPRKK-GCWKCGKEGHQMKDCT ERQANFLGKIWPSYKG-RPGNFLQSRPEPTAPPFLQSRPEPTAPPEESLRS GVETTTPSQKQEPIDKELYPLTSLRSLFGNDPSSQ

6. A process according to claim I, characterized in that a host cell is transformed with an expression vector capable of expressing a polypeptide with the amino acid sequence: PIVQNIQGQMVHQAISPRTLNAWVKVVEEKAFSPE-VIPMFSALSEGATPQ DLNTMLNTVGGHQAAMQM-LKETINEEAAEWDRVHPVHAGPIAPGQMREPR GSDIAGTTSTLQEQIGWMTNNPPIPVGEIYKRWIIL-GLNKIVRMYSPTSI LDIRQGPKEPFRDYVDRFYKT-LRAEQASQEVKNWMTETLLVQNANPDCKT ILKAL-GPAATLEEMMTACQGVGGPGHK

7. A process according to any one of claims I to 6, characterized in that as a host cell a yeast cell is used.

8. A process according to claim 7, characterized in that as a yeast cell a S. cerevisiae cell is used.

9. A process for producing polypeptides immunologically equivalent to the gag-protein products of HTLV-III comprising: transforming a yeast cell with an expression vector comprising a gene coding for said gag-protein products operably linked to a promoter sequence enabling transcription, translation and expression of said gag-protein products in said yeast cell; culturing said yeast cell so that the protein products are expressed; and, extracting and isolating said protein products.

10. A process for the preparation of an expression vector capable in a host cell of effecting the expression of a polypeptide as defined in any one of claims I to 6, which process comprises isolating a gene coding for said polypeptides and operably linking said gene with a promoter sequence.

II. A process according to claim I0, characterized in that a promoter sequence capable of effecting expression in a yeast cell is used.

I2. A process according to claim II, characterized in that a promoter sequence capable of effecting expression in S. cerevisiae cell is used.

I3. A process according to claim I2, characterized in that a PHO5 promoter sequence is used.

I4. A process according to any one of claims I0 to I3, characterized in that as a gene the ClaI to EcoRI restriction size fragment of the λHXB-3 genome is used.

I5. A process according to any one of claims I0 to I3, characterized in that as a gene the ClaI to BglII restriction site fragment of the ClaI to EcoRI restriction site fragment of the λHXB-3 genome is used.

I6. A process according to any one of claims I0 to I3, characterized in that as a gene the ClaI to EcoRI restriction site fragment of λHXB-3, wherein the ClaI to EcoRI fragment is filled in at the BclI restriction site with the base sequence GATC, is used.

I7. A process for the preparation of an expression vector capable in a yeast cell of effecting the expression of polypeptides immunologically equivalent to the gag-protein products of HTLV-III, which process comprises isolating a gene coding for said polypeptides and operably linking said gene with a promoter sequence.

I8. A process for the preparation of a transformed cell carrying an expression vector capable in a host cell of effecting the expression of a polypeptide as defined in any one of claims I to 6, which process comprises transforming a host cell with said expression vector by methods known in the art.

I9. A process according to claim I8, characterized in that as a host cell a yeast cell is used.

20. A process according to claim I9, characterized in that as a yeast cell a S. cerevisiae cell is used.

21. A process according to claim 20, characterized in that as a S. cerevisiae cell S. cerevisiae 20B-I2 is used.

22. A process for the preparation of a transformed yeast cell carrying an expression vector capable in a host cell of effecting the expression of polypeptides immunologically equivalent to the gag-protein products of HTLV-III, which process comprises transforming a yeast cell with said expression vector by methods known in the art.

23. A process for testing human blood for the presence of antibodies to the viral etiologic agent of AIDS which method comprises mixing a composition containing a polypeptide as defined in any one of claims I to 6 or 9 or mixtures thereof with a sample of human blood and determining whether said protein or any of its natural proteolytic proteins or mixtures thereof binds to AIDS antibodies present in the blood sample.

24. A process for the preparation of a vaccine comprising mixing a polypeptide as defined in any one of claims I to 6 or 9 with a physiologically acceptable carrier.

25. A process for the preparation of antibodies against AIDS virus comprising injecting a mammalin or avian animal with a sufficient amount of a polypeptide as defined in any one of claims I to 6 or 9 and recovering said antibodies from the serum of said animals.

26. Antibodies raised against a polypeptid as defined in any one of claims I to 6 or 9.

27. The antibodies of claim 26 which are monoclonal antibodies.

28. A polypeptide immunoloqically equivalent to the gag-protein products of HTLV-III whenever prepared by a process as claimed in any one of claims I to 9.

29. A gene containing a gene portion having a DNA sequence encoding a polypeptide as defined in any one of claims I to 6 operably linked to a promoter capable of effecting the expression of said DNA sequence.

30. The gene of claim 29 wherein said gene portion is the ClaI to EcoRI restriction site fragment of the λ HXB-3 genome.

3I. The gene of claim 30 wherein the promoter is the Bam HI to Aha III restriction site fragment of PHO5.

32. The gene of claim 29 wherein said gene portion is the ClaI to BglII restriction site fragment of the ClaI to EcoRI fragment of the λ HXB-3 genome.

33. The gene of claim 32 wherein the promoter is the BamHI to Aha III restriction site fragment of PHO5.

34. The gene of claim 29 where the gene portion is the ClaI to EcoRI restriction site fragment of λ HXB-3, wherein the ClaI to EcoRI fragment is filled in at its BclI restriction site with the base sequence GATC.

35. The gene of claim 34 wherein the promoter is the BamHI to AhaIII restriction site fragment of PHO5.

36. A gene containing a gene portion having a DNA sequence encoding the gag-protein products of HTLV-III operably linked to a promoter capable of effecting the expression of said DNA sequence in yeast.

37. A recombinant expression vector capable of effecting the expression of a polypeptide as defined in any one of claims I to 6 containing a gene portion having a DNA sequence encoding said polypeptide operably linked to a promoter capable of effecting the expression of said DNA sequence.

38. A vector according to claim 37 wherein the gene portion is the ClaI to EcoRI restriction fragment of the λ HXB-3 genome.

39. A vector according to claim 38 wherein the promoter is the BamHI to AhaIII restriction site fragment of PHO5.

40. A vector according to claim 37 wherein the gene portion is the ClaI to BglII restriction site fragment of the Cla I to EcoRI restriction site fragment of the λ HXB-3 genome.

4I. A vector according to claim 40 wherein the promoter is the BamHI to AhaIII restriction site fragment of PHO5.

42. A vector according to claim 37 wherein the gene portion is the ClaI to EcoRI restriction site fragment of λ HXB-3, wherein the ClaI to EcoRI fragment is filled in at the BclI restriction site with the base sequence GATC.

43. A vector according to claim 42 wherein the promoter is the BamHI to AhaIII restriction site fragment of PHO5.

44. A vector according to claim 39 which is pYE72/gagI.

45. A vector according to claim 4I which is pYE72/gag2.

46. A vector according to claim 43 which is pYE72/gag3.

47. A recombinant expression vector capable of effecting the expression of the gag-protein products of HTLV-III in yeast containing a gene portion having a DNA sequence encoding the gag-protein of HTLV-III operably linked to a promoter capable of effecting the expression of said DNA sequence.

48. A transformed cell carrying a vector as claimed in any one of claims 37 to 46.

49. A transformed cell according to claim 48 which is a yeast cell.

50. A transformed cell according to claim 49 which is a S. cereviiae yeast cell.

5I. A transformed cell according to claim 50 which is S. cerevisiae 20B-I2.

52. The use of a polypeptide as defined in any one of claims l to 6 or 9 for the preparation of a protective immunization vaccine.

53. The use of a polypeptide as defined in any one of claims l to 6 or 9 for the preparation of antibodies against AIDS virus.

54. The use of a polypeptide as defined in any one of claims l to 6 or 9 for testing human blood for the presence of AIDS virus.

55. A test kit for the determination of antibodies against AIDS virus comprising in a container a polypeptide as defined in any one of claims l to 6 or 9.

56. A test kit for the determination of AIDS virus comprising in a container antibodies against AIDS virus elicited by a polypeptide as defined in any one of claims l to 6 or 9.

Figure 1-A

Figure 1–B

*BamH* I  *Aha* III  *Pst* I

*Aha* III
ACCA ATG TTT AAA
TGGT TAC AAA TTT
Met Phe Lys  **PHO5**

*EcoR* I  *Cla* I  *Bgl* II *Bcl* I  *EcoR* I

36bp  *Cla* I
ATG ········· GAT CGA TGG
TAC ········· CTA GCT ACC
Met ········· Asp Arg Trp  **gag/pol**

*BamH* I/*Aha* III
Isolate 560bp fragment

*Cla* I
Klenow
*EcoR* I
Isolate 3900bp fragment

Ligate
*BamH* I/*EcoR* I

*BamH* I  *Bgl* II *Bcl* I  *EcoR* I

ACCA ATG TTT CGA TGG
Met Phe Arg Trp

Figure 2

Figure 3

FIGURE 4

BglII

1 GAGAGACAGGCTAATTTTTTAGGGAAGATCTGGCCTTCCTACAAGGGAAGGCCAGGGAATTTTCTTCAG 69
gag - GluArgGlnAlaAsnPheLeuGlyLysIleTrpProSerTyrLysGlyArgProGlyAsnPheLeuGln
pol -        PhePheArgGluAspLeuAlaPheLeuGlnGlyLysAlaArgGluPheSerSerG

70 AGCAGACCAGAGCCAACAGCCCCACCATTTCTTCAGAGCAGACCAGAGCCAACAGCCCCACCAGAAGAG 138
SerArgProGluProThrAlaProProPheLeuGlnSerArgProGluProThrAlaProProGluGlu
luGlnThrArgAlaAsnSerProThrIleSerSerGluGlnThrArgAlaAsnSerProThrArgArgG

139 AGCTTCAGGTCTGGGGTAGAGACAACAACTCCCCCTCAGAAGCAGGAGCCGATAGACAAGGAACTGTAT 207
SerPheArgSerGlyValGluThrThrThrProProGlnLysGlnGluProIleAspLysGluLeuTyr
luLeuGlnValTrpGlyArgAspAsnAsnSerProSerGluAlaGlyAlaAspArgGlnGlyThrValS

208 CCTTTAACTTCCCTCAGATCACTCTTTGGCAACGACCCCTCGTCACAATAAAGATAGGGGGGCAACTAA 276
ProLeuThrSerLeuArgSerLeuPheGlyAsnAspProSerSerGln   .
erPheAsnPheProGlnIleThrLeuTrpGlnArgProLeuValThrIleLysIleGlyGlyGlnLeuL

277 AGGAAGCTCTATTAGATACAGGAGCAGATGATACAGTATTAGAAGAAATGAGTTTGCCAGGAAGATGGA 345

ysGluAlaLeuLeuAspThrGlyAlaAspAspThrValLeuGluGluMETSerLeuProGlyArgTrpL

0 230 222

Figure 5

0 230 222

Figure 5 (cont.)

BclI

346 AACCAAAAATGATAGGGGGAATTGGAGGTTTTATCAAAGTAAGACAGTATGATCAGATACTCATAGAAA 414

    ysProLysMETIleGlyGlyIleGlyGlyPheIleLysValArgGlnTyrAspGlnIleLeuIleGluI

                                                 ArgSerAspThrHisArgAs

415 TCTGTGGACATAAAGCTATAGGTACAGTATTAGTAGGACCTACACCTGTCAACATAATTGGAA 477

    leCysGlyHisLysAlaIleGlyThrValLeuValGlyProThrProValAsnIleIleGly

    nLeuTrpThr .

Figure 6-A

p14 —
p16 —
p24 —
p56 —

— 14K
— 18K
25K —
43K —
68K —

East

Figure 6-B

0 230 222

—p14
—p16

—p24

—p56

—14K
—18K

—25K

—43K

—68K

West

## FIGURE 7

```
     GAG 56 SEQ
ATGTTTCGATGGGAAAAAATTCGGTTAAGGCCAGGGGGAAAGAAAAAATATAAATTAAAACA
TATAGTATGGGCAAGCAGGGAGCTAGAACGATTCGCAGTTAATCCTGGCCTGTTAGAAACAT
CAGAAGGCTGTAGACAAATACTGGGACAGCTACAACCATCCCTTCAGACAGGATCAGAAGAA
CTTAGATCATTATATAATACAGTAGCAACCCTCTATTGTGTGCATCAAAGGATAGAGATAAA
AGACACCAAGGAAGCTTTAGACAAGATAGAGGAAGAGCAAAACAAAAGTAAGAAAAAAGCAC
AGCAAGCAGCAGCTGACACAGGACACAGCAGTCAGGTCAGCCAAAATTACCCTATAGTGCAG
AACATCCAGGGGCAAATGGTACATCAGGCCATATCACCTAGAACTTTAAATGCATGGGTAAA
AGTAGTAGAAGAGAAGGCTTTCAGCCCAGAAGTAATACCCATGTTTTCAGCATTATCAGAAG
GAGCCACCCCACAAGATTTAAACACCATGCTAAACACAGTGGGGGGACATCAAGCAGCCATG
CAAATGTTAAAAGAGACCATCAATGAGGAAGCTGCAGAATGGGATAGAGTACATCCAGTGCA
TGCAGGGCCTATTGCACCAGGCCAGATGAGAGAACCAAGGGGAAGTGACATAGCAGGAACTA
CTAGTACCCTTCAGGAACAAATAGGATGGATGACAAATAATCCACCTATCCCAGTAGGAGAA
ATTTATAAAAGATGGATAATCCTGGGATTAAATAAAATAGTAAGAATGTATAGCCCTACCAG
CATTCTGGACATAAGACAAGGACCAAAAGAACCCTTTAGAGACTATGTAGACCGGTTCTATA
AAACTCTAAGAGCCGAGCAAGCTTCACAGGAGGTAAAAAATTGGATGACAGAAACCTTGTTG
GTCCAAAATGCGAACCCAGATTGTAAGACTATTTTAAAAGCATTGGGACCAGCAGCTACACT
AGAAGAAATGATGACAGCATGTCAGGGAGTAGGAGGACCCGGCCATAAGGCAAGAGTTTTGG
CTGAAGCAATGAGCCAAGTAACAAATACAGCTACCATAATGATGCAGAGAGGCAATTTTAGG
AACCAAAGAAAGATTGTTAAGTGTTTCAATTGTGGCAAAGAAGGGCACATAGCCAGAAATTG
CAGGGCCCCTAGGAAAAAGGGCTGTTGGAAATGTGGAAAGGAAGGACACCAAATGAAAGATT
GTACTGAGAGACAGGCTAATTTTTTAGGGAAGATCTGGCCTTCCTACAAGGGAAGGCCAGGG
AATTTTCTTCAGAGCAGACCAGAGCCAACAGCCCCACCATTTCTTCAGAGCAGACCAGAGCC
AACAGCCCCACCAGAAGAGAGCCTCAGGTCTGGGGTAGAGACAACAACTCCCTCTCAGAAGC
AGGAGCCGATAGACAAGGAACTGTATCCTTTAACTTCCCTCAGATCACTCTTTGGCAACGAC
CCCTCGTCACAATAA
```

# FIGURE 8

GAG56.PEP
P56
MFRWEKIRLRPGGKKKYKLKHIVWASRELERFAVNPGLLETSEGCRQILG
QLQPSLQTGSEELRSLYNTVATLYCVHQRIEIKDTKEALDKIEEEQNKSK
KKAQQAAADTGHSSQVSQNYPIVQNIQGQMVHQAISPRTLNAWVKVVEEK
AFSPEVIPMFSALSEGATPQDLNTMLNTVGGHQAAMQMLKETINEEAAEW
DRVHPVHAGPIAPGQMREPRGSDIAGTTSTLQEQIGWMTNNPPIPVGEIY
KRWIILGLNKIVRMYSPTSILDIRQGPKEPFRDYVDRFYKTLRAEQASQE
VKNWMTETLLVQNANPDCKTILKALGPAATLEEMMTACQGVGGPGHKARV
LAEAMSQVTNTATIMMQRGNFRNQRKIVKCFNCGKEGHIARNCRAPRKKG
CWKCGKEGHQMKDCTERQANFLGKIWPSYKGRPGNFLQSRPEPTAPPFLQ
SRPEPTAPPEESLRSGVETTTPSQKQEPIDKELYPLTSLRSLFGNDPSSQ

## FIGURE 9

```
GAG24.SEQ
CCTATAGTGCAGAACATCCAGGGGCAAATGGTACATCAGGCCATATCACCTAGAACTTTAAA
TGCATGGGTAAAAGTAGTAGAAGAGAAGGCTTTCAGCCCAGAAGTAATACCCATGTTTTCAG
CATTATCAGAAGGAGCCACCCCACAAGATTTAAACACCATGCTAAACACAGTGGGGGGACAT
CAAGCAGCCATGCAAATGTTAAAAGAGACCATCAATGAGGAAGCTGCAGAATGGGATAGAGT
ACATCCAGTGCATGCAGGGCCTATTGCACCAGGCCAGATGAGAGAACCAAGGGGAAGTGACA
TAGCAGGAACTACTAGTACCCTTCAGGAACAAATAGGATGGATGACAAATAATCCACCTATC
CCAGTAGGAGAAATTTATAAAAGATGGATAATCCTGGGATTAAATAAAATAGTAAGAATGTA
TAGCCCTACCAGCATTCTGGACATAAGACAAGGACCAAAAGAACCCTTTAGAGACTATGTAG
ACCGGTTCTATAAAACTCTAAGAGCCGAGCAAGCTTCACAGGAGGTAAAAAATTGGATGACA
GAAACCTTGTTGGTCCAAAATGCGAACCCAGATTGTAAGACTATTTTAAAAGCATTGGGACC
AGCAGCTACACTAGAAGAAATGATGACAGCATGTCAGGGAGTAGGAGGACCCGGCCATAAG
```

# FIGURE 10

GAG24.PEP

P24

PIVQNIQGQMVHQAISPRTLNAWVKVVEEKAFSPEVIPMFSALSEGATPQ
DLNTMLNTVGGHQAAMQMLKETINEEAAEWDRVHPVHAGPIAPGQMREPR
GSDIAGTTSTLQEQIGWMTNNPPIPVGEIYKRWIILGLNKIVRMYSPTSI
LDIRQGPKEPFRDYVDRFYKTLRAEQASQEVKNWMTETLLVQNANPDCKT
ILKALGPAATLEEMMTACQGVGGPGHK

## FIGURE 11

GAG16.SEQ
ATGCAGAGAGGCAATTTTAGGAACCAAAGAAAGATTGTTAAGTGTTTCAATTGTGGCAAAGA
AGGGCACATAGCCAGAAATTGCAGGGCCCCTAGGAAAAAGGGCTGTTGGAAATGTGGAAAGG
AAGGACACCAAATGAAAGATTGTACTGAGAGACAGGCTAATTTTTTAGGGAAGATCTGGCCT
TCCTACAAGGGAAGGCCAGGGAATTTTCTTCAGAGCAGACCAGAGCCAACAGCCCCACCATT
TCTTCAGAGCAGACCAGAGCCAACAGCCCCACCAGAAGAGAGCCTCAGGTCTGGGGTAGAGA
CAACAACTCCCTCTCAGAAGCAGGAGCCGATAGACAAGGAACTGTATCCTTTAACTTCCCTC
AGATCACTCTTTGGCAACGACCCCTCGTCACAA

# FIGURE 12

GAG16.PEP

P16
MQRGNFRNQRKIVKCFNCGKEGHIARNCRAPRKKGCWKCGKEGHQMKDCT
ERQANFLGKIWPSYKGRPGNFLQSRPEPTAPPFLQSRPEPTAPPEESLRS
GVETTTPSQKQEPIDKELYPLTSLRSLFGNDPSSQ

## FIGURE 13

```
GAG14.SEQ
ATGTTTCGATGGGAAAAAATTCGGTTAAGGCCAGGGGGAAAGAAAAAATATAAATTAAAACA
TATAGTATGGGCAAGCAGGGAGCTAGAACGATTCGCAGTTAATCCTGGCCTGTTAGAAACAT
CAGAAGGCTGTAGACAAATACTGGGACAGCTACAACCATCCCTTCAGACAGGATCAGAAGAA
CTTAGATCATTATATAATACAGTAGCAACCCTCTATTGTGTGCATCAAAGGATAGAGATAAA
AGACACCAAGGAAGCTTTAGACAAGATAGAGGAAGAGCAAAACAAAAGTAAGAAAAAAGCAC
AGCAAGCAGCAGCTGACACAGGACACAGCAGTCAGGTCAGCCAAAATTAC
```

# FIGURE 14

GAG14.PEP

P14

MFRWEKIRLRPGGKKKYKLKHIVWASRELERFAVNPGLLETSEGCRQILG
QLQPSLQTGSEELRSLYNTVATLYCVHQRIEIKDTKEALDKIEEEQNKSK
KKAQQAAADTGHSSQVSQNY

## FIGURE 15

GAG48.SEQ

```
ATGTTTCGATGGGAAAAAATTCGGTTAAGGCCAGGGGGGAAAGAAAAAATATAAATTAAAACA
TATAGTATGGGCAAGCAGGGAGCTAGAACGATTCGCAGTTAATCCTGGCCTGTTAGAAACAT
CAGAAGGCTGTAGACAAATACTGGGACAGCTACAACCATCCCTTCAGACAGGATCAGAAGAA
CTTAGATCATTATATAATACAGTAGCAACCCTCTATTGTGTGCATCAAAGGATAGAGATAAA
AGACACCAAGGAAGCTTTAGACAAGATAGAGGAAGAGCAAAACAAAAGTAAGAAAAAAGCAC
AGCAAGCAGCAGCTGACACAGGACACAGCAGTCAGGTCAGCCAAAATTACCCTATAGTGCAG
AACATCCAGGGGCAAATGGTACATCAGGCCATATCACCTAGAACTTTAAATGCATGGGTAAA
AGTAGTAGAAGAGAAGGCTTTCAGCCCAGAAGTAATACCCATGTTTTCAGCATTATCAGAAG
GAGCCACCCCACAAGATTTAAACACCATGCTAAACACAGTGGGGGGACATCAAGCAGCCATG
CAAATGTTAAAAGAGACCATCAATGAGGAAGCTGCAGAATGGGATAGAGTACATCCAGTGCA
TGCAGGGCCTATTGCACCAGGCCAGATGAGAGAACCAAGGGGAAGTGACATAGCAGGAACTA
CTAGTACCCTTCAGGAACAAATAGGATGGATGACAAATAATCCACCTATCCCAGTAGGAGAA
ATTTATAAAAGATGGATAATCCTGGGATTAAATAAAATAGTAAGAATGTATAGCCCTACCAG
CATTCTGGACATAAGACAAGGACCAAAAGAACCCTTTAGAGACTATGTAGACCGGTTCTATA
AAACTCTAAGAGCCGAGCAAGCTTCACAGGAGGTAAAAAATTGGATGACAGAAACCTTGTTG
GTCCAAAATGCGAACCCAGATTGTAAGACTATTTTAAAAGCATTGGGACCAGCAGCTACACT
AGAAGAAATGATGACAGCATGTCAGGGAGTAGGAGGACCCGGCCATAAGGCAAGAGTTTTGG
CTGAAGCAATGAGCCAAGTAACAAATACAGCTACCATAATGATGCAGAGAGGCAATTTTAGG
AACCAAAGAAAGATTGTTAAGTGTTTCAATTGTGGCAAAGAAGGGCACATAGCCAGAAATTG
CAGGGCCCCTAGGAAAAAGGGCTGTTGGAAATGTGGAAAGGAAGGACACCAAATGAAAGATT
GTACTGAGAGACAGGCTAATTTTTTAGGGAAGATCCACAGGACGGGTGTGGTCGCCATGATC
GCGTAG
```

# FIGURE 16

GAG48.PEP

P48

```
MFRWEKIRLRPGGKKKYKLKHIVWASRELERFAVNPGLLETSEGCRQILG
QLQPSLQTGSEELRSLYNTVATLYCVHQRIEIKDTKEALDKIEEEQNKSK
KKAQQAAADTGHSSQVSQNYPIVQNIQGQMVHQAISPRTLNAWVKVVEEK
AFSPEVIPMFSALSEGATPQDLNTMLNTVGGHQAAMQMLKETINEEAAEW
DRVHPVHAGPIAPGQMREPRGSDIAGTTSTLQEQIGWMTNNPPIPVGEIY
KRWIILGLNKIVRMYSPTSILDIRQGPKEPFRDYVDRFYKTLRAEQASQE
VKNWMTETLLVQNANPDCKTILKALGPAATLEEMMTACQGVGGPGHKARV
LAEAMSQVTNTATIMMQRGNFRNQRKIVKCFNCGKEGHIARNCRAPRKKG
CWKCGKEGHQMKDCTERQANFLGKIHRTGVVAMIA
```

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | PROCEEDINGS OF THE NATIONAL ACADEMY OF SCIENCES OF THE USA, vol. 82, no. 22, November 1985, pages 7748-7752, Washington, US; D.J. DOWBENKO et al.: "Bacterial expression of the acquired immunodeficiency syndrome retrovirus p24 gag protein and its use as a diagnostic reagent" * Pages 7749-7751 * | 1,6,8, 16,34, 38-40 | C 12 N   15/00<br>C 07 K   13/00<br>C 12 N    1/18<br>A 61 K   39/21<br>C 12 P   21/02<br>G 01 N   33/569<br>C 07 K   15/00 |
| X | SCIENCE, vol. 228, 31st May 1985, pages 1094-1096; F. BARIN et al.: "Virus envelope protein of HTLV-III represents major target antigen for antibodies in AIDS patients" * Figure 1 * | 1,2,5, 6,8,38 -40 | |
| X | SCIENCE, vol. 228, 3rd May 1985, pages 593-595; W.G. ROBEY et al.: "Characterization of envelope and core structural gene products of HTLV-III with sera from AIDS patients" * Figure 1 *  ---                -/- | 1,2,6, 8,38- 40 | **TECHNICAL FIELDS SEARCHED (Int. Cl.4)**<br><br>C 12 N<br>A 61 K<br>G 01 N |

The present search report has been drawn up for all claims

| Place of search | Date of completion of the search | Examiner |
|---|---|---|
| THE HAGUE | 06-04-1987 | CUPIDO M. |

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl.4) |
|---|---|---|---|
| X | NATURE, vol. 313, 7th February 1985, pages 450-458; M.A. MUESING et al.: "Nucleic acid structure and expression of the human AIDS/lymphadenopathy retrovirus"<br>* Page 452, last paragraph; page 453 * | 1,2,6,8,38-40 | |
| X | PROCEEDINGS OF THE NATIONAL ACADEMY OF SCIENCES OF THE USA, vol. 82, August 1985, pages 5199-5202; F. DI MARZO VERONESE et al.: "Monoclonal antibodies specific for p24, the major core protein of human T-cell leukemia virus type III"<br>* Page 5201 * | 6,8,40-44 | |
| X,P | EP-A-0 181 150 (CHIRON CORP.)<br>* Pages 30,31 * | 1,5,6,8 | TECHNICAL FIELDS SEARCHED (Int. Cl.4) |
| X,P | SCIENCE, vol. 231, 28th March 1986, pages 1580-1584; R.A. KRAMER et al.: "HTLV-III gag protein is precessed in yeast cells by the virus pol-protease"<br>* Whole article * | 1-46 | |
| X,P | EP-A-0 187 041 (GENENTECH INC.)<br>* Claims 1-3,10-12,15,16 * | 1,8 | |

The present search report has been drawn up for all claims

| Place of search | Date of completion of the search | Examiner |
|---|---|---|
| THE HAGUE | 06-04-1987 | CUPIDO M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82